# EUROPÄISCHE PATENTSCHRIFT

(11) **EP 3 963 309 B1**
(45) Veröffentlichungstag und Bekanntmachung des Hinweises auf die Patenterteilung: **15.11.2023**
(21) Anmeldenummer: 20723807.2
(22) Anmeldetag: 27.04.2020
(51) Int. Cl.: G01N 21/3504, G01N 21/31, A61M 16/00, A61M 16/08

(54) **GEGEN MECHANISCHE STÖREINFLÜSSE KOMPENSIERTER MEHRKANAL-IR-GASSENSOR**
MULTICHANNEL IR GAS SENSOR THAT IS COMPENSATED AGAINST MECHANICAL DISTURBANCES
CAPTEUR DE GAZ INFRAROUGE MULTICANAUX COMPENSÉ CONTRE DES PERTURBATIONS MÉCANIQUES

(30) Priorität: 03.05.2019 DE 102019111563
(43) Veröffentlichungstag der Anmeldung: 09.03.2022
(73) Patentinhaber: Hamilton Medical AG, 7402 Bonaduz (CH)
(72) Erfinder: SCHRANZ, Christoph, 7402 Bonaduz (CH); NOVOTNI, Dominik, 7000 Chur (CH)
(74) Vertreter: Ruttensperger Lachnit Trossin Gomoll
(86) Internationale Anmeldenummer: PCT/EP2020/061644
(87) Internationale Veröffentlichungsnummer: WO 2020/225010

(56) Entgegenhaltungen:
- EP-A1- 1 482 301
- US-A1- 2001 015 408

## Beschreibung

Die vorliegende Erfindung betrifft einen Mehrkanal-Infrarot-Gassensor gemäß dem Oberbegriff von Anspruch 1. Ein derartiger Gassensor ist aus der US 2001/0015408 A1 bekannt und umfasst:
- eine Strahlenteileranordnung, welche dazu ausgebildet ist, einen auf die Strahlenteileranordnung längs einer vorbestimmten Einstrahlachse einstrahlenden Infrarotstrahl in eine Mehrzahl von Infrarot-Teilstrahlen zu teilen,
- einen in einem ersten Strahlengang eines ersten Infrarot-Teilstrahls angeordneten ersten Bandfilter mit einer vorbestimmten ersten Bandbreite und mit einem Transmissionsmaximum bei einer vorbestimmten ersten Nutzsignal-Wellenlänge,
- einen im ersten Strahlengang des ersten Infrarot-Teilstrahls hinter dem ersten Bandfilter angeordneten ersten Infrarot-Nutzsignal-Sensor,
- einen zweiten Bandfilter, welcher in einem zweiten Strahlengang eines vom ersten Infrarot-Teilstrahl verschiedenen zweiten Infrarot-Teilstrahls angeordnet ist, wobei der zweite Bandfilter eine vorbestimmte zweite Bandbreite und ein Transmissionsmaximum bei einer vorbestimmten ersten Referenzsignal-Wellenlänge aufweist, wobei die erste Referenzsignal-Wellenlänge von der ersten Nutzsignal-Wellenlänge verschieden ist,
- einen im zweiten Strahlengang des zweiten Infrarot-Teilstrahls hinter dem zweiten Bandfilter gelegenen ersten Infrarot-Referenzsignal-Sensor.

Die vorliegende Erfindung betrifft weiter eine Beatmungsvorrichtung mit einem solchen Mehrkanal-Infrarot-Gassensor.

Der Begriff "Infrarot" ist in der vorliegenden Anmeldung auch als IR abgekürzt. Ebenso wird der Mehrkanal-Infrarot-Gassensor nachfolgend auch als "Mehrkanal-IR-Gassensor" oder nur kurz als "Gassensor" bezeichnet.

Ein weiterer Gassensor ist aus der US 2007/0241280 A1 bekannt. Aus dieser Druckschrift ist es ebenfalls bekannt, einen solchen Gassensor zur Messung eines Gasanteils, insbesondere CO₂, in einem Beatmungsgas eines lebenden Patienten zu verwenden.

Bei der künstlichen Beatmung von lebenden Patienten, sei es nun eine künstliche Beatmung eines vollständig sedierten oder komatösen und damit zu eigener Atmung unfähigen Patienten oder sei es zur lediglich unterstützenden Beatmung eines wenigstens zeitabschnittsweise spontan atmenden Patienten, ist die Kenntnis von Gasbestandteilen des Beatmungsgases zur Überwachung der vitalen Funktionen des Patienten oder/und zur Überwachung des korrekten Betriebs der Beatmungsvorrichtung hilfreich und wichtig. Dies ist im einschlägigen Fachgebiet hinreichend bekannt. So kann beispielsweise durch Erfassung des CO₂-Anteils im inspiratorischen Beatmungsgas und im exspiratorischen Beatmungsgas ermittelt werden, wie gut die Verstoffwechselung von Sauerstoff im Patienten funktioniert. Dies soll jedoch nur eines von mehreren möglichen Beispielen sein.

Der aus der US 2007/0241280 A1 bekannte Gassensor verwendet einen sowohl reflektierenden als auch transmittierenden Strahlenteiler, um einen in den Strahlenteiler einstrahlenden Infrarotstrahl in zwei Infrarot-Teilstrahlen zu teilen. Ein erster Infrarot-Teilstrahl wird durch einen ersten Bandfilter auf einen ersten Sensor gelenkt. Dieser erste Bandfilter weist ein Transmissionsmaximum bei der Infrarot-Absorptions-Wellenlänge von CO₂ als der Nutzsignal-Wellenlänge auf und weist eine kleine erste Bandbreite auf, damit sich das Sensorsignal in Abhängigkeit vom jeweiligen CO₂-Gehalt des von dem in den Gassensor einstrahlenden Infrarotstrahl durchstrahlten Messgases möglichst stark ändert. Der erste Sensor ist somit ein Infrarot-Nutzsignal-Sensor.

Ein zweiter Infrarot-Teilstrahl wird nach Durchgang durch einen zweiten Bandfilter und zusätzlich durch einen Kerbfilter auf einen zweiten Sensor gelenkt. Der zweite Bandfilter hat ein Transmissionsmaximum bei der Infrarot-Absorptions-Wellenlänge von CO₂ als der Referenzsignal-Wellenlänge und weist eine größere Bandbreite als der erste Bandfilter auf. Der Kerbfilter hat ein Auslöschungsmaximum bzw. ein Transmissionsminimum ebenfalls bei der Infrarot-Absorption-Wellenlänge von CO₂. Die Folge ist, dass sich das Signal des zweiten Sensors mit der Änderung des CO₂-Gehalts des Messgases nicht oder nur in vernachlässigbarem Umfang ändert. Der zweite Sensor ist folglich ein Infrarot-Referenzsignal-Sensor.

Der IR-Referenzsignal-Sensor ist erforderlich, um durch Vergleich des Signals des IR-Nutzsignal-Sensors mit dem Signal des IR-Referenzsignal-Sensors das Ausmaß an Absorption von Infrarotlicht durch CO₂ im Messgas und damit den Anteil von CO₂ im Messgas beurteilen zu können. Dies gilt auch für die vorliegende Erfindung.

Dabei ist es hilfreich, das Nutzsignal des IR-Nutzsignal-Sensors einerseits und das Referenzsignal des IR-Referenzsignal-Sensors aus ein und demselben einstrahlenden Infrarotstrahl abzuleiten, um sicherzustellen, dass sowohl das Nutzsignal als auch das Referenzsignal sowohl qualitativ als auch quantitativ im Wesentlichen denselben Störfaktoren ausgesetzt war, sodass eine lediglich durch Störfaktoren bewirkte quantitative Änderung des Nutzsignals auch eine entsprechende Änderung des Referenzsignals bewirkt. Dadurch kann verhindert werden, dass aus einer Änderung des Nutzsignals irrtümlich auf eine Änderung des durch die Nutzsignal-Wellenlänge identifizierten Gasanteils geschlossen wird. Auch dies gilt für die vorliegende Erfindung.

An dem bekannten Gassensor sind der IR-Nutzsignal-Sensor und der IR-Referenzsignal-Sensor derart angeordnet, dass ihre jeweiligen Sensor-Erfassungsflächen um eine virtuelle Neigeachse aufeinander zu geneigt sind. In den in der der US 2007/0241280 A1 gezeigten schematischen Darstellungen sind die jeweiligen ebenen Sensor-Erfassungsflächen relativ zueinander um 90° geneigt orientiert. Die allseits verlängert gedachten Sensor-Erfassungsflächen des bekannten Gassensor schneiden sich in ihrer gemeinsamen virtuellen Neigeachse. Die Sensor-Erfassungsfläche des IR-Nutzsignal-Sensors und die Sensor-Erfassungsfläche des IR-Referenzsignal-Sensors sind bezüglich einer die virtuelle Neigeachse enthaltenden Symmetrieebene spiegelsymmetrisch angeordnet.

Die US 2007/0241280 A1 nennt keine konkreten IR-Sensoren, die im bekannten Gassensor zum Einsatz kommen. Es gibt jedoch IR-Sensoren, die nicht nur sensitiv für Einstrahlung von Infrarotlicht sind, sondern auch sensitiv für mechanische Belastungen, wie etwa Erschütterungen. Derartige mechanische sensitive IR-Sensoren sind überdies richtungsabhängig mechanisch sensitiv, d. h. eine betragsgleiche mechanische Belastung wirkt sich auf ein und denselben IR-Sensor abhängig von der Richtung, aus der sie auf den IR-Sensor einwirkt, unterschiedlich aus.

Nachteilig an dem aus der US 2007/0241280 A1 bekannten Gassensor ist daher, dass eine mechanische Belastung, die einheitlich auf den Gassensor insgesamt einwirkt, sich in unterschiedlicher Weise auf den IR-Nutzsignal-Sensor und den IR-Referenzsignal-Sensor auswirkt und somit eine unerwünschte Ungenauigkeit in der Bestimmung des interessierenden Gasanteils im Messgas bewirken kann.

Dies gilt auch für den aus der US 2001/0015408 A1 bekannten Gassensor, welcher einen pyramidonalen Strahlteiler, welcher einen eintreffenden IR-Strahl in vier Teilstrahlen teilt, welche in einer gemeinsamen Ebene liegen und von welchen je zwei in Umfangsrichtung um die Einfallsrichtung benachbarte Teilstrahlen zwischen sich einen rechten Winkel einschließen. Zwei IR-Nutzsignal-Sensoren liegen sich über den Strahlteiler hinweg mit sowohl zueinander als auch zur Einfallsrichtung parallelen Nutzsignal-Sensor-Erfassungsflächen ebenso gegenüber, wie zwei IR-Referenzsignal-Sensoren. Somit sind die Nutzsignal-Teilstrahlen kollinear, aber breiten sich in entgegengesetzte Richtungen aus und sind die Referenzsignal-Teilstrahlen kollinear, aber breiten sich in entgegengesetzte Richtungen aus.

Nur der Vollständigkeit halber sei auf die EP 1 482 301 A1 verwiesen, welche einen IR-Gassensor mit zwei IR-Nutzsignal-Sensoren und mit zwei IR-Referenzsignal-Sensoren zur Verwendung an einer Beatmungsvorrichtung offenbart. Der aus der EP 1 482 301 A1 bekannte Gassensor verwendet jedoch einen halbdurchlässigen Spiegel als Strahlteiler, so dass bei diesem abweichend vom gattungsgemäßen Mehrkanal-IR-Gassensor die Verlaufsrichtungen des ersten, des zweiten, des dritten und des vierten Strahlengangs strahlenabwärts des Strahlteilers nicht paarweise voneinander verschieden sind.

Es ist daher Aufgabe der vorliegenden Erfindung, den eingangs genannten Gassensor hinsichtlich seiner mechanischen Sensitivität derart zu verbessern, dass er bei gleicher mechanischer Belastung wie der Gassensor des Standes der Technik eine höhere Genauigkeit liefert bzw. sein Erfassungsergebnis durch mechanische Belastung weniger stark beeinflussbar ist als das Erfassungsergebnis des Gassensors des Standes der Technik.

Diese Aufgabe wird erfindungsgemäß gelöst durch einen Gassensor mit allen Merkmalen des Anspruchs 1. Bei diesem ist, wie schon beim Mehrkanal-Infrarot-Gassensor der US 2001/0015408 A1 die Strahlenteileranordnung dazu ausgebildet, den einstrahlenden Infrarotstrahl in wenigstens vier Infrarot-Teilstrahlen zu teilen, wobei der Mehrkanal-Infrarot-Gassensor weiter umfasst:
- einen dritten Bandfilter, welcher in einem dritten Strahlengang eines dritten Infrarot-Teilstrahls angeordnet ist, wobei der dritte Bandfilter eine vorbestimmte dritte Bandbreite und ein Transmissionsmaximum bei einer vorbestimmten zweiten Nutzsignal-Wellenlänge aufweist,
- einen im dritten Strahlengang des dritten Infrarot-Teilstrahls hinter dem dritten Bandfilter gelegenen zweiten Infrarot-Nutzsignal-Sensor,
- einen vierten Bandfilter, welcher in einem vierten Strahlengang eines vierten Infrarot-Teilstrahls angeordnet ist, wobei der vierte Bandfilter eine vorbestimmte vierte Bandbreite und ein Transmissionsmaximum bei einer vorbestimmten zweiten Referenzsignal-Wellenlänge aufweist,
- einen im vierten Strahlengang des vierten Infrarot-Teilstrahls hinter dem vierten Bandfilter gelegenen zweiten Infrarot-Referenzsignal-Sensor,
wobei die Verlaufsrichtungen des ersten, des zweiten, des dritten und des vierten Strahlengangs bzw. der Infrarot-Teilstrahlen paarweise voneinander verschieden sind, wobei jede Wellenlänge aus der ersten und der zweiten Referenzsignal-Wellenlänge von jeder Wellenlänge aus der ersten und der zweiten Nutzsignal-Wellenlänge verschieden ist, wobei der erste und der zweite Infrarot-Nutzsignal-Sensor derart angeordnet sind, dass ihre jeweiligen Nutzsignal-Sensor-Erfassungsflächen bezüglich einer zwischen den Nutzsignal-Sensor-Erfassungsflächen gelegenen Nutzsignal-Sensor-Symmetrieebene symmetrisch orientiert sind, und wobei der erste und der zweite Infrarot-Referenzsignal-Sensor derart angeordnet sind, dass ihre jeweiligen Referenzsignal-Sensor-Erfassungsflächen bezüglich einer zwischen den Referenzsignal-Sensor-Erfassungsflächen gelegenen Referenzsignal-Sensor-Symmetrieebene symmetrisch orientiert sind, wobei keine Nutzsignal-Sensor-Erfassungsfläche orthogonal zur Nutzsignal-Sensor-Symmetrieebene orientiert ist und wobei keine Referenzsignal-Sensor-Erfassungsfläche orthogonal zur Referenzsignal-Sensor-Symmetrieebene orientiert ist.

Wenn in der vorliegenden Anmeldung im Zusammenhang mit der Erfindung allgemein von IR-Sensoren die Rede ist, so sind damit sowohl die IR-Nutzsignal- wie auch die IR-Referenzsignal-Sensoren bezeichnet. Sind jeweils nur beide IR-Nutzsignal-Sensoren oder nur beide IR-Referenzsignal-Sensoren angesprochen, ohne dass es auf die Bestimmung des jeweiligen IR-Sensorenpaars als Nutzsignal- oder Referenzsignal-Sensoren ankäme, ist in der vorliegenden Anmeldung von bestimmungsgleichen IR-Sensoren die Rede.

Durch Teilung des in die Strahlenteileranordnung einstrahlenden Infrarotstrahls in wenigstens vier Infrarot-Teilstrahlen können wenigstens zwei Infrarot-Sensoren als IR-Nutzsignal-Sensoren und wenigstens zwei Infrarot-Sensoren als IR-Referenzsignal-Sensoren verwendet werden. Die jeweils wenigstens zwei bestimmungsgleichen Infrarot-Sensoren sind erfindungsgemäß relativ zueinander symmetrisch orientiert. Somit kann sich eine zur Symmetrieebene zweier bestimmungsgleicher Infrarot-Sensoren, also zur Nutzsignal-Sensor-Symmetrieebene der IR-Nutzsignal-Sensoren einerseits und zur Referenzsignal-Sensor-Symmetrieebene der IR-Referenzsignal-Sensoren andererseits, orthogonale mechanische Belastung auf die beiden symmetrisch orientierten IR-Sensoren mit gleichem oder ähnlichem Wirkungsbetrag aber entgegengesetzten Wirkungsrichtungen auswirken. Durch geeignete Schaltung oder/und Auswertung der Sensorsignale der beiden symmetrisch orientierten IR-Sensoren können die betragsgleichen oder betragsähnlichen aber entgegengesetzt gerichteten unerwünschten Wirkungen der mechanischen Belastung, etwa eines Stoßes, zur gegenseitigen Auslöschung oder Reduzierung verwendet werden. Obwohl jeder einzelne IR-Sensor ein durch die mechanische Belastung unerwünscht verändertes Sensorsignal liefert, kann die unerwünschte Wirkung der mechanischen Belastung auf die Signale beider bestimmungsgleicher IR-Sensoren ausschließlich auf Grundlage der Signale selbst in einfacher Weise reduziert oder sogar beseitigt werden. Dies kann beispielsweise durch Bildung eines geometrischen oder arithmetischen Mittelwerts aus den einzelnen Signalen bestimmungsgleicher Sensoren erreicht werden. Dies gilt für die IR-Nutzsignal-Sensoren untereinander ebenso wie für die IR-Referenzsignal-Sensoren untereinander. Somit ist eine Kompensation des erfindungsgemäßen Gassensors gegenüber mechanischen Störeinflüssen aus wenigstens einer Raumrichtung ohne zusätzliche Filter allein aus den Signalen selbst aufgrund der beschriebenen Orientierung und Anordnung der IR-Sensoren möglich.

"Paarweise verschieden" bedeutet dabei, dass je zwei beliebig ausgewählte Infrarot-Teilstrahlen unterschiedliche Verlaufsrichtungen im Strahlengang aufweisen. Dies kann einen Abschnitt des Strahlengangs betreffen, betrifft jedoch vorzugsweise den gesamten Strahlengang der wenigstens vier Infrarot-Teilstrahlen nach Erreichen der Strahlenteileranordnung.

Durch Orientierung der Sensor-Erfassungsflächen bestimmungsgleicher IR-Sensoren nicht-orthogonal zu ihrer zugeordneten Symmetrieebene kann eine verhältnismäßig große Sensor-Erfassungsfläche und damit hohe Gassensorempfindlichkeit erhalten werden, ohne dass sich hieraus ein Gassensor mit großer Grundfläche orthogonal zur Einstrahlachse ergibt. Hierdurch kann gerade für die weiter unten angesprochenen Beatmungsvorrichtungen ein vorteilhaft räumlich kompakter Gassensor erhalten werden.

Es wird im Verlauf der vorliegenden Anmeldung erläutert werden, wie durch weitere vorteilhafte Maßnahmen mechanische Störeinflüsse auch aus anderen Raumrichtungen wenigstens teilweise kompensiert werden können, sodass in Weiterbildung des zuvor beschriebenen erfindungsgemäßen Gassensors ein zunehmend umfangreich gegenüber mechanischen Störeinflüssen unempfindlicher Gassensor mit einfachen Mitteln erhalten werden kann.

Wenngleich der Gassensor mehr als vier IR-Teilstrahlen nutzen kann, teilt die Strahlenteileranordnung den einstrahlenden Infrarotstrahl bevorzugt in genau vier Infrarot-Teilstrahlen, die nach Durchgang durch Bandfilter auf IR-Sensoren treffen.

Es gilt zu beachten, dass die vorliegende Erfindung keine symmetrische Anordnung bestimmungsgleicher IR-Sensoren bezüglich ihrer Symmetrieachse fordert, aber auch nicht ausschließt, sondern lediglich eine symmetrische Orientierung der Sensor-Erfassungsflächen bestimmungsgleicher IR-Sensoren bezüglich ihrer Symmetrieebene verlangt. Da es für eine Auswirkung einer mechanischen Belastung auf den Gassensor nur auf die Wirkungsrichtung der mechanischen Belastung ankommt, ist die oben genannte symmetrische Orientierung von Sensor-Erfassungsflächen jeweils bestimmungsgleicher IR-Sensoren bezüglich der ihnen zugeordneten Sensor-Symmetrieebene nicht in dem Sinne streng symmetrisch zu verstehen, dass ein IR-Sensor seinem bestimmungsgleichen IR-Sensor über die beiden IR-Sensoren zugeordnete Sensor-Symmetrieebene gegenüberliegen muss. Es reicht zur Erfüllung der hier geforderten Orientierungs-Symmetrie aus, wenn die Sensor-Erfassungsflächen bestimmungsgleicher IR-Sensoren symmetrisch nicht-orthogonal zu der ihnen zugeordneten Symmetrieebene orientiert sind, wenn also die Sensor-Erfassungsflächen beider bestimmungsgleichen IR-Sensoren entweder parallel zu ihrer Symmetrieebene angeordnet sind oder auf unterschiedlichen Seiten der Symmetrieebene um den gleichen Winkel bezüglich einer gemeinsamen, in der zugeordneten Symmetrieebene liegenden virtuellen Neigeachse, jedoch in unterschiedlichen Neigungsrichtungen geneigt angeordnet sind. Die symmetrisch orientierten Sensor-Erfassungsflächen können längs einer in der jeweiligen zugeordneten Symmetrieebene liegenden Achse zueinander versetzt angeordnet sein.

Wegen der sich daraus ergebenden Vereinfachung der Strahlenführung der Infrarot-Teilstrahlen nach Erreichen der Strahlenteileranordnung sind jedoch bevorzugt bestimmungsgleiche IR-Sensoren mit ihren jeweiligen Sensor-Erfassungsflächen nicht nur symmetrisch bezüglich ihrer zugeordneten Symmetrieebene orientiert, sondern bezüglich ihrer Symmetrieebene symmetrisch angeordnet und liegen einander bevorzugt über die jeweilige Symmetrieebene hinweg gegenüber.

Die Symmetrie der Orientierung bestimmungsgleicher IR-Sensoren ist wegen der sich daraus ergebenden Vereinfachung der Strahlungsführung der wenigstens vier Infrarot-Teilstrahlen bevorzugt eine Spiegelsymmetrie. Somit sind die Nutzsignal-Sensor- und die Referenzsignal-Sensor-Symmetrieebene bevorzugt jeweils Spiegelsymmetrieebenen.

Bevorzugt sind der erste und der zweite IR-Nutzsignal-Sensor sowie der erste und der zweite IR-Referenzsignal-Sensor jeweils pyroelektrische Infrarot-Sensoren. Pyroelektrische Infrarot-Sensoren weisen in der Regel eine beschichtete Sensor-Erfassungsfläche auf, deren Beschichtung sich durch Einstrahlung von Infrarotlicht erwärmt. Die meisten pyroelektrischen Infrarot-Sensoren weisen auch piezoelektrische Eigenschaften auf und sind daher sensitiv für mechanische Belastungen. Da die piezoelektrischen Effekte an pyroelektrischen Infrarot-Sensoren in der Regel nur für orthogonal zur Sensor-Erfassungsfläche wirkende mechanische Belastungskomponenten auftreten, dagegen nicht für mechanische Belastungskomponenten parallel zur Sensor-Erfassungsfläche, sind die bevorzugten pyroelektrischen Infrarot-Sensoren als die IR-Sensoren des Gassensors der vorliegenden Erfindung richtungsanisotrop mechanisch sensitiv.

Bevorzugt sind die IR-Nutzsignal-Sensoren und die IR-Referenzsignal-Sensoren jeweils baugleiche, vorzugsweise identische Sensoren. Die Bestimmung eines verwendeten IR-Sensors als IR-Nutzsignal-Sensor oder als IR-Referenzsignal-Sensor ist somit von dem mit dem jeweiligen IR-Sensor zusammenwirkenden Bandfilter abhängig. Die Wellenlänge des Transmissionsmaximums des jeweiligen Bandfilters entscheidet vorrangig darüber, ob der hinter dem jeweiligen Bandfilter angeordnete IR-Sensor ein IR-Nutzsignal- oder ein IR-Referenzsignal-Sensor ist. Man wird die erste und die zweite Referenzsignal-Wellenlänge so wählen, dass sie möglichst nicht mit einer Absorptions-Wellenlänge eines Gasbestandteils des mit dem Gassensor zu messenden Messgases übereinstimmt. Dann ist das von den IR-Referenzsignal-Sensoren erhaltene Signal in erwünschter Weise im Wesentlichen unabhängig von der Zusammensetzung des Messgases.

Wenigstens eine Wellenlänge aus der ersten und der zweiten Nutzsignal-Wellenlänge wird dagegen bevorzugt so ausgewählt, dass sie mit einer Absorptions-Wellenlänge des vom Gassensor zu erfassenden Gasbestandteils des Messgases übereinstimmt, oder zumindest so nahe an dieser Absorptions-Wellenlänge liegt, dass eine Änderung des Anteils des zu erfassenden Gasbestandteils am Messgas zu einer signifikanten Änderung des Nutzsignals der IR-Nutzsignal-Sensoren führt. Der vorliegend vorgestellte Mehrkanal-IR-Gassensor ist bevorzugt ein nicht-dispersiver CO2- oder ein nicht-dispersiver NOx-, insbesondere ein nicht-dispersiver NO2-Sensor.

Ganz grundsätzlich können die Nutzsignal-Sensor- und die Referenzsignal-Sensor-Symmetrieebene in einer identischen Symmetrieebene zusammenfallen. In diesem Falle liegen auf jeder Seite dieser Symmetrieebene je ein IR-Nutzsignal-Sensor und ein IR-Referenzsignal-Sensor. Dabei müssen sich, wie oben geschildert, bestimmungsgleiche IR-Sensoren nicht unmittelbar über die Symmetrieebene hinweg gegenüberliegen, werden dies wegen der sich daraus ergebenden Einfachheit der Strahlenführung der Infrarot-Teilstrahlen jedoch in der Regel tun. Ein vorteilhaft räumlich kompakter Gassensor kann erhalten werden, wenn die Referenzsignal-Sensor-Symmetrieebene von der Nutzsignal-Sensor-Symmetrieebene verschieden ist. Insbesondere vorteilhaft ist wegen der dadurch erhältlichen räumlichen Kompaktheit des Gassensors bei gleichzeitig verfügbarem verhältnismäßig großem Bauraum zur Anordnung der einzelnen IR-Sensoren eine zueinander orthogonale Anordnung der Nutzsignal-Sensor-Symmetrieebene und der Referenzsignal-Sensor-Symmetrieebene.

Wie oben bereits geschildert wurde, können die Sensor-Erfassungsflächen bestimmungsgleicher IR-Sensoren parallel zu der ihnen zugeordneten Symmetrieebene orientiert sein. Dies kann jedoch die Strahlführung der vier Infrarot-Teilstrahlen auf die jeweiligen Sensor-Erfassungsflächen verkomplizieren. Erfindungsgemäß ist daher vorgesehen, dass der erste und der zweite Infrarot-Nutzsignal-Sensor derart angeordnet sind, dass ihre jeweiligen Sensor-Erfassungsflächen um eine virtuelle Nutzsignal-Neigeachse aufeinander zu geneigt sind, und dass der erste und der zweite Infrarot-Referenzsignal-Sensor derart angeordnet sind, dass ihre jeweiligen Sensor-Erfassungsflächen um eine von der Nutzsignal-Neigeachse verschiedene virtuelle Referenzsignal-Neigeachse aufeinander zu geneigt sind.

Bei der beschriebenen aufeinander zu geneigten Anordnung kann bei der am häufigsten auftretenden piezoelektrischen Eigenschaft eines IR-Sensors orthogonal zur seiner Sensor-Erfassungsfläche ein weiterer Kompensationseffekt genutzt werden: die Komponente einer mechanischen Belastung orthogonal zur Symmetrieebene eines Paars bestimmungsgleicher IR-Sensoren kann, wie oben bereits beschrieben, wegen ihrer betragsgleichen oder betragsähnlichen aber entgegengesetzt gerichteten Signalbeiträge in jedem der bestimmungsgleichen IR-Sensoren durch gegenseitige Auslöschung oder wenigstens Reduzierung kompensiert werden. Eine weitere Komponente der mechanischen Belastung in der Symmetrieebene und orthogonal zur virtuellen Neigeachse führt in beiden bestimmungsgleichen IR-Sensoren zu einem sowohl betragsgleichen als auch richtungsgleichen Signalbeitrag der mechanischen Belastung. Dann, wenn sowohl die IR-Nutzsignal-Sensoren als auch die IR-Referenzsignal-Sensoren in gleicher Weise um jeweils eine Nutzsignal-Neigeachse bzw. Referenzsignal-Neigeachse geneigt sind und die beiden unterschiedlichen Neigeachsen entweder in einer gemeinsamen oder in zueinander parallelen Ebenen liegen, sind die Komponenten der Signalbeiträge der mechanischen Belastung in der jeweiligen Symmetrieebene und orthogonal zur jeweiligen Neigeachse für alle IR-Sensoren gleich groß, gleich gerichtet und treten synchron auf. Somit ist zwar jedes Signal eines jeden IR-Sensors durch die mechanische Belastung verändert, jedoch jedes Signal eines jeden IR-Sensors in gleicher Weise mit gleichem Betrag und gleichzeitig, sodass auch dieser Einfluss der mechanischen Belastung bei Berücksichtigung von Referenzsignal und Nutzsignal zur Ermittlung eines Anteils einer Gaskomponente im Messgas erheblich verringert oder sogar vollständig kompensiert werden kann.

Ebenso vorteilhaft kann zur möglichst geringen Bauraumbeanspruchung des Gassensors die Strahlenteileranordnung eine Reflektor-Strahlenteileranordnung sein. Dies bedeutet, dass die wenigstens vier Infrarot-Teilstrahlen alle durch Reflexion des einstrahlenden Infrarotstrahls von der Reflektor-Strahlenteileranordnung in unterschiedliche Richtungen gebildet werden. Durch Einsatz einer solchen Reflektor-Strahlenteileranordnung können die vier durch Reflexion gebildeten Infrarot-Teilstrahlen sehr einfach auf die zuvor beschriebenen um jeweilige Neigeachsen aufeinander zu geneigten Sensor-Erfassungsflächen der IR-Nutzsignal-Sensoren und der IR-Referenzsignal-Sensoren gelenkt werden. Bei Verwendung einer Reflektor-Strahlenteileranordnung können sich alle Infrarot-Nutzsignal-Sensoren sowie alle Infrarot-Referenzsignal-Sensoren auf ein und derselben Seite der Reflektor-Strahlenteileranordnung befinden, etwa auf einer Einstrahlseite der Strahlenteileranordnung, auf welcher der einstrahlende Infrarotstrahl auf die Strahlenteileranordnung einstrahlt. Somit kann im Wesentlichen die gesamte Optik und Sensorik des Mehrkanal-Infrarot-Gassensors zwischen einer Einstrahlöffnung oder einem Einstrahlfenster, durch welche hindurch ein Infrarotstrahl auf die Strahlenteileranordnung einstrahlt und einem Träger der Reflektor-Strahlenteileranordnung angeordnet sein.

Die Strahlenteileranordnung kann mehrere Teilanordnungen umfassen, von welchen jede einen auf sie einstrahlenden Infrarotstrahl in weniger als vier Infrarot-Teilstrahlen teilt, welche aber insgesamt für einen eingangs einstrahlenden Infrarotstrahl vier Infrarot-Teilstrahlen liefern. Die oben bevorzugte Reflektor-Strahlenteileranordnung kann jedoch in einfacher Weise eine Mehrzahl von Reflektorkörpern aufweisen, welche jeweils eine Anzahl unterschiedlich orientierter Reflektorflächen aufweisen, und zwar bevorzugt für jeden gewünschten Infrarot-Teilstrahl je eine. Die Reflektorkörper können also polyedrische sich längs einer Verjüngungsachse verjüngende Körper sein, deren Seitenflächen Reflektorflächen für den einstrahlenden Infrarotstrahl sind. Beispielsweise können dann, wenn genau vier Infrarot-Teilstrahlen gewünscht sind, pyramidonale Reflektorkörper verwendet werden, wobei bevorzugt, wegen des sich daraus ergebenden symmetrischen Aufbaus des Gassensors, die Verjüngungsachsen der pyramidonalen Reflektorkörper jeweils parallel zur Einstrahlachse ausgerichtet sind.

Mit insgesamt geringem Bauraumbedarf, aber dennoch ausreichend Raum zur Anordnung verhältnismäßig großer Sensor-Erfassungsflächen können die Infrarot-Nutzsignal-Sensoren und die Infrarot-Referenzsignal-Sensoren alternierend in Umfangsrichtung um eine virtuelle Anordnungsachse angeordnet sind.

Ein einfacher Aufbau, bei welchem die oben genannten sich verjüngenden polyedrischen Reflektorkörper verwendet werden können, ermöglicht eine bauraumsparende Anordnung, bei welcher der erste und der zweite Infrarot-Nutzsignal-Sensor einander bezüglich der virtuellen Anordnungsachse diametral gegenüberliegen oder/und bei welcher der erste und der zweite Infrarot-Referenzsignal-Sensor einander bezüglich der virtuellen Anordnungsachse diametral gegenüberliegen.

Dann, wenn die virtuelle Anordnungsachse die Einstrahlachse ist, kann der Gassensor mit einer einzigen Umlenkung eines jeden der wenigstens vier Infrarot-Teilstrahlen bezüglich des einstrahlenden Infrarotstrahls auskommen. Hierdurch wird der Infrarotstrahl als Informationsträger in nur sehr geringem Maße durch die Strahlteilung beeinflusst, was ein genaues Erfassungsergebnis des Gassensors ermöglicht.

Eine möglichst gleichmäßige Bestrahlung der IR-Sensoren und ihrer Sensor-Erfassungsflächen mit den Infrarot-Teilstrahlen in dem Sinne, dass auf jede Sensor-Erfassungsfläche der wenigstens vier IR-Sensoren ein Infrarot-Teilstrahl mit im Wesentlichen gleicher Intensität einfällt, kann dadurch erhalten werden, dass die Nutzsignal-Sensor-Symmetrieebene oder/und die Referenzsignal-Sensor-Symmetrieebene die Anordnungsachse enthält bzw. enthalten oder parallel zur Anordnungsachse verläuft bzw. verlaufen. Bevorzugt ist die Anordnungsachse die Schnittgerade der Nutzsignal-Sensor-Symmetrieebene und der Referenzsignal-Sensor-Symmetrieebene

Bevorzugt sind die jeweils bezüglich einer Sensor-Symmetrieebene symmetrisch orientierten bestimmungsgleichen IR-Sensoren derart zu ihrer Sensor-Symmetrieebene orientiert, dass ihre jeweilige Sensor-Erfassungsfläche mit der Sensor-Symmetrieebene einen Winkel mit einem Betrag von 45° einschließt. Somit können zur Sensor-Symmetrieebene orthogonale mechanische Belastungen unmittelbar durch Additive oder subtraktive Signalverarbeitung der bestimmungsgleichen IR-Sensoren ausgelöscht oder stark gemindert werden. Beispielsweise können die Signale bestimmungsgleicher IR-Sensoren durch Mittelwertbildung zu einer gemeinsamen Signalinformation verarbeitet werden. Als Mittelwert kann ein geometrischer oder ein arithmetischer Mittelwert gebildet werden. Wenn die Sensor-Symmetrieebene die Anordnungsachse enthält, gilt dies für die bezüglich der Sensor-Symmetrieebene symmetrisch orientierten bestimmungsgleichen IR-Sensoren für jede zur Anordnungsachse orthogonale mechanische Belastung. Bevorzugt enthalten daher sowohl die Nutzsignal-Sensor-Symmetrieebene als auch die Referenzsignal-Sensor-Symmetrieebene die Anordnungsachse.

Um zu erreichen, dass beide bestimmungsgleichen IR-Sensoren durch eine mechanische Belastung mit einer Komponente orthogonal zur jeweiligen Neigeachse des Paars bestimmungsgleicher IR-Sensoren in gleicher Weise belastet werden, kann die Nutzsignal-Neigeachse oder die Referenzsignal-Neigeachse orthogonal zur Anordnungsachse verlaufen. Bevorzugt verlaufen sowohl die Nutzsignal- als auch die Referenzsignal-Neigeachse orthogonal zur Anordnungsachse, damit sich eine parallel zur Anordnungsachse verlaufende Komponente einer mechanischen Belastung auf alle vier IR-Sensoren synchron, betragsgleich und gleichgerichtet auswirkt. Somit kann sichergestellt werden, dass sich das durch die beiden IR-Referenzsignal-Sensoren bereitgestellte Referenzsignal in gleicher Weise und gleichzeitig durch die längs der Anordnungsachse verlaufende Komponente der mechanischen Belastung verändert wie das durch die beiden IR-Nutzsignal-Sensoren bereitgestellte Nutzsignal.

Eine mechanische Belastungskomponente parallel zur Neigeachse bzw. orthogonal zur Anordnungsachse und parallel zur zugeordneten Symmetrieebene eines Paars bestimmungsgleicher IR-Sensoren hat in der Regel keine Auswirkungen auf das vom IR-Sensorpaar gelieferte Signal. Somit kann der Gassensor nahezu vollständig, also unabhängig von der Wirkungsrichtung einer auf ihn einwirkenden mechanischen Belastung, gegenüber mechanischen Belastungen unempfindlich gemacht werden.

Zur Erzielung einer möglichst geringen Anzahl an Umlenkungen des einstrahlenden Infrarotstrahls bzw. der sich aus ihm ergebenden Infrarot-Teilstrahlen ist es vorteilhaft, wenn die Sensor-Erfassungsfläche jedes Infrarot-Nutzsignal-Sensors und die Sensor-Erfassungsfläche jedes Infrarot-Referenzsignal-Sensors zur Strahlenteileranordnung hin geneigt ist.

Grundsätzlich kann daran gedacht sein, dass jede der Referenzsignal-Wellenlängen größer als jede der Nutzsignal-Wellenlängen oder kleiner als jede der Nutzsignal-Wellenlängen sind. Grundsätzlich können auch beide Referenzsignal-Wellenlängen identisch sein. Eine besonders hohe Qualität des Referenzsignals kann man jedoch dann erhalten, wenn die beiden Referenzsignal-Wellenlängen betragsmäßig verschieden sind, da dann Zufallsstörungen durch eine vorübergehend unerwartet vorhandene Gaskomponente Messgas sich nur auf einen der beiden IR-Referenzsignal-Sensoren auswirken. Zur Bereitstellung eines möglichst robusten Referenzsignals ist die erste oder die zweite Nutzsignal-Wellenlänge betragsmäßig zwischen der ersten und der zweiten Referenzsignal-Wellenlänge gelegen. Besonders bevorzugt sind beide Nutzsignal-Wellenlängen zwischen der ersten und der zweiten Referenzsignal-Wellenlänge gelegen.

Die Nutzsignal-Wellenlängen definieren vom Gassensor erfassbare Gaskomponenten im Messgas. Grundsätzlich kann daran gedacht sein, dass sich die Nutzsignal-Wellenlängen betragsmäßig unterscheiden, obwohl sie dieselbe Gaskomponente erfassen sollen. Um sicherstellen zu können, dass die IR-Nutzsignal-Sensoren den auf sie einfallenden Infrarot-Teilstrahl hinsichtlich der von diesem transportierten Absorptionsinformation optimal auswerten können, ist es vorteilhaft, wenn die erste und die zweite Nutzsignal-Wellenlänge sich betragsmäßig um nicht mehr als ein Drittel der betragsmäßig kleineren Bandbreite aus erster und dritter Bandbreite unterscheiden. Bevorzugt sind die erste und die zweite Nutzsignal-Wellenlänge betragsmäßig gleich. Ein bevorzugter Anwendungsfall des vorliegend beschriebenen Gassensors ist die Erfassung von CO₂ in inspiratorischem oder/und exspiratorischem Beatmungsgas während einer apparativen Beatmung eines Patienten. Die erste und die zweite Nutzsignal-Wellenlänge liegen zur CO₂-Erfassung bevorzugt in einem Bereich zwischen 4,25 µm und 4,28 µm. Die erste Referenzsignal-Wellenlänge kann im Bereich von 3,90 µm bis 4,0 µm, besonders bevorzugt bei 3,95 µm liegen, die zweite Referenzsignal-Wellenlänge kann im Bereich von 4,40 µm bis 4,5 µm, besonders bevorzugt bei 4,45 µm liegen.

Um dafür zu sorgen, dass das Signal der IR-Nutzsignal-Sensoren empfindlich auf Änderungen des Anteils der durch die Nutzsignal-Wellenlänge in definierten Gaskomponente reagiert, während das Signal der IR-Referenzsignal-Sensoren möglichst konstant bleiben soll, ist es vorteilhaft, wenn jede Bandbreite aus zweiter und vierter Bandbreite betragsmäßig kleiner ist als jede Bandbreite aus erster und dritter Bandbreite. So können die zweiten und vierten Bandfilter jeweils eine Bandbreite im zweistelligen Nanometerbereich aufweisen und können der erste und der dritte Bandfilter jeweils eine Bandbreite im dreistelligen Nanometerbereich aufweisen. Von dem zweiten und dem vierten Bandfilter kann, ebenso wie vom ersten und dritten Bandfilter, derjenige Bandfilter mit der kleineren Transmissionsmaximum-Wellenlänge die größere Bandbreite aufweisen. Bandbreiten für den zweiten und vierten Bandfilter liegen bevorzugt in einem Bereich von 50 bis 99 nm, insbesondere in einem Bereich von 60 bis 90 nm. Bandbreiten für den ersten und dritten Bandfilter liegen bevorzugt im Bereich von 150 bis 200 nm, insbesondere in einem Bereich von 170 bis 180 nm.

Der oben beschriebene Gassensor bietet aufgrund der vorteilhaften Orientierung und Anordnung von IR-Nutzsignal-Sensoren und IR-Referenzsignal-Sensoren die grundsätzliche Möglichkeit, den Gassensor unempfindlich gegenüber mechanischen äußeren Belastungen auszubilden. Eine tatsächliche Signalkompensation der im Gassensor verwendeten Signale kann dadurch erhalten werden, dass der Gassensor eine Auswertevorrichtung umfasst, welche aus den Signalen des ersten und des zweiten Infrarot-Referenzsignal-Sensors eine Referenzinformation ermittelt, welche aus den Signalen des ersten und des zweiten Infrarot-Nutzsignal-Sensors eine Nutzinformation ermittelt, und welche aus einem Vergleich von Referenzinformation und Nutzinformation eine Information über einen Anteil eines durch die erste oder/und die zweite Nutzsignal-Wellenlänge identifizierten Gases an einem mit dem in den Gassensor eintretenden Infrarotstrahl bestrahlten Messgas ausgibt. Die Auswertevorrichtung kann durch einen integrierten Schaltkreis oder Mikrochip in einem Sensorgehäuse des Gassensors realisiert sein. Die Auswertevorrichtung kann jedoch auch eine äußere Auswertevorrichtung sein, welche außerhalb des Sensorgehäuses des Gassensors angeordnet ist und mit den IR-Sensoren des Gassensors in signalübertragender Leitungsverbindung steht.

Gerade bei der künstlichen Beatmung werden die von Beatmungsgas durchströmten Leitungsbauteile während des Beatmungsbetriebs verschmutzt. Dies gilt vor allem für die exspiratorische Leitungsseite, die von Körperflüssigkeiten des Patienten erreicht wird. Ein häufiger Austausch der Messküvette, die ebenfalls durch Feuchtigkeit, Speichel und dergleichen verunreinigt werden kann, ist daher vorteilhaft. Um den mit teurer Messtechnik versehenen Gassensor mit austauschbaren Messküvetten verwenden zu können, kann der Gassensor ein Sensorgehäuse aufweisen mit einem ersten Kompartiment, in welchem die Strahlenteileranordnung, die Infrarot-Nutzsignal-Sensoren und die Infrarot-Referenzsignal-Sensoren angeordnet sind, und mit einem räumlich vom ersten Kompartiment entfernt gelegenen zweiten Kompartiment, in welchem eine Infrarot-Strahlenquelle angeordnet ist, wobei zwischen dem ersten und dem zweiten Kompartiment eine Aufnahmeformation zur Aufnahme einer Messküvette zwischen dem ersten und dem zweiten Kompartiment angeordnet ist. Die Aufnahmeformation kann einen Aufnahmespalt zwischen den beiden Kompartimenten umfassen, in welchen ein Abschnitt einer vom zweiten Kompartiment zum ersten Kompartiment hin durchstrahlbaren Messküvette aufnehmbar ist.

Wegen der besonders vorteilhaften Einsetzbarkeit des vorliegend beschriebenen Gassensors betrifft die vorliegende Erfindung außerdem eine Beatmungsvorrichtung zur wenigstens unterstützend künstlichen Beatmung eines lebenden Patienten, umfassend:
- eine Beatmungsgasquelle,
- eine Beatmungsleitungsanordnung, um inspiratorisches Beatmungsgas von der Beatmungsgasquelle zu einer patientenseitigen proximalen Beatmungsgas-Auslassöffnung und um exspiratorisches Beatmungsgas von einer proximalen Beatmungsgas-Einlassöffnung weg zu leiten,
- eine Druckveränderungsvorrichtung zur Veränderung des Drucks des Beatmungsgases in der Beatmungsleitungsanordnung,
- eine Steuervorrichtung zum Betrieb der Beatmungsgasquelle oder/und der Druckveränderungsvorrichtung sowie
- einen Mehrkanal-Infrarot-Gassensor, wie er oben beschrieben und weitergebildet ist, zur Erfassung wenigstens eines Gasbestandteils im inspiratorischen oder/und exspiratorischen Beatmungsgas.

Eine Beatmungsgasquelle kann dabei ein Beatmungsgasspeicher sein, etwa eine Gasflasche, oder die Beatmungsgasquelle kann eine Kopplungsformation zur strömungsmechanischen Kopplung mit einer Hausinstallation einer Klinik sein, welche in einem Leitungsnetz Beatmungsgas bereitstellt. Die Beatmungsgasquelle kann auch ein Gebläse sein, welche aus einem Beatmungsgasreservoir Beatmungsgas entnimmt und in der Beatmungsleitungsanordnung fördert. Das Beatmungsgasreservoir kann in diesem Fall die Umgebung der Beatmungsvorrichtung sein, aus welchem die Beatmungsgasquelle Umgebungsluft entnimmt.

Die Druckveränderungsvorrichtung kann ein Ventil umfassen, beispielsweise zur Druckminderung eines aus der Beatmungsgasquelle entspannten Beatmungsgases. Die Druckveränderungsvorrichtung kann das zuvor genannte Gebläse sein oder umfassen. Je nach Ausgestaltung der Beatmungsgasquelle können also die Beatmungsgasquelle und die Druckveränderungsvorrichtung auch durch übereinstimmende Vorrichtungsbestandteile zweier Vorrichtungen oder durch ein und dieselbe Vorrichtung gebildet sein. Eine Messküvette kann Beatmungsgas führen, welches vom Gassensor zur Erfassung des Vorhandenseins wenigstens einer Gaskomponente und ihres Anteils am Messgas mit Infrarot bestrahlt bzw. durchleuchtet wird. Die Messküvette kann eine Nebenstrom-Messküvette sein, in welcher Beatmungsgas strömt, das von der Beatmungsleitungsanordnung abgezweigt wurde.

Vorteilhafterweise umfasst die Beatmungsleitungsanordnung eine von Beatmungsgas durchströmbare Messküvette, welche einen abzweigungsfreien Teil der Beatmungsleitungsanordnung bildet und mit dem Mehrkanal-Infrarot-Gassensor zur Infrarot-Durchstrahlung von Beatmungsgas koppelbar ist. Dann ist die Messküvette eine Hauptstrom-Messküvette, welche unmittelbar das dem Patienten zugeleitete inspiratorische oder/und das vom Patienten weg geleitete exspiratorische Beatmungsgas führt. Vorteilhafterweise ist die Messküvette in der Beatmungsleitungsanordnung lösbar und austauschbar angeordnet, um sie bei entsprechender Verschmutzung gegen eine funktionierende neue Messküvette einfach, schnell und hygienisch austauschen zu können.

Die oben beschriebene Auswertevorrichtung des Gassensors kann Teil der Steuervorrichtung der Beatmungsvorrichtung sein.

Vorteilhafterweise ist die Steuervorrichtung dazu ausgebildet einen oder mehrere Betriebsparameter der Beatmungsgasquelle, etwa wenn diese ein Gebläse ist, oder/und der Druckveränderungsvorrichtung auf Grundlage der Erfassungsergebnisse des Mehrkanal-Infrarot-Gassensors zu verändern.

Die Beatmungsgas-Auslassöffnung und die Beatmungsgas-Einlassöffnung können ein und dieselbe Öffnung sein, beispielsweise am proximalen Ende eines Endotrachealtubus. Es können aber auch zwei verschiedene Öffnungen sein. Die Beatmungsvorrichtung kann eine beliebige Patientenschnittstelle aufweisen, etwa den zuvor genannten Endotrachealtubus oder eine Larynxmaske oder eine andere für eine künstliche Beatmung taugliche Schnittstelle.

Die vorliegende Erfindung wird nachfolgend anhand der beiliegenden Zeichnungen näher erläutert werden. Es stellt dar:
- Fig.1: eine schematische Explosionsdarstellung einer erfindungsgemäßen Beatmungsvorrichtung,
- Fig.2: eine grobschematische Querschnittsdarstellung durch die Messküvette von Figur 1 mit dem daran aufgenommenen erfindungsgemäßen Mehrkanal-Infrarot-Gassensor von Figur 1 im Längsschnitt,
- Fig.3: eine Draufsicht auf die Strahlenteileranordnung, die vier Infrarot-Sensoren und die vier von den Infrarot-Sensoren teilweise verdeckten Bandfilter von Figur 2 bei Betrachtung längs der Einstrahlachse ausgehend von der Schnittebene III-III in Figur 2,
- Fig. 4: eine grobschematische Erläuterung der kompensierenden Wirkung der Anordnung von Infrarot-Sensoren im Mehrkanal-Infrarot-Gassensor, und
- Fig. 5: ein beispielhaftes Diagramm mit den unterschiedlichen Signalpegeln der IR- Nutzsignal-Sensoren während Phasen einer Absorption, Nicht-Absorption und wieder Absorption von CO₂ (oben) und mit den Signalpegeln der IR-Referenzsignal-Sensoren während derselben Phasen.

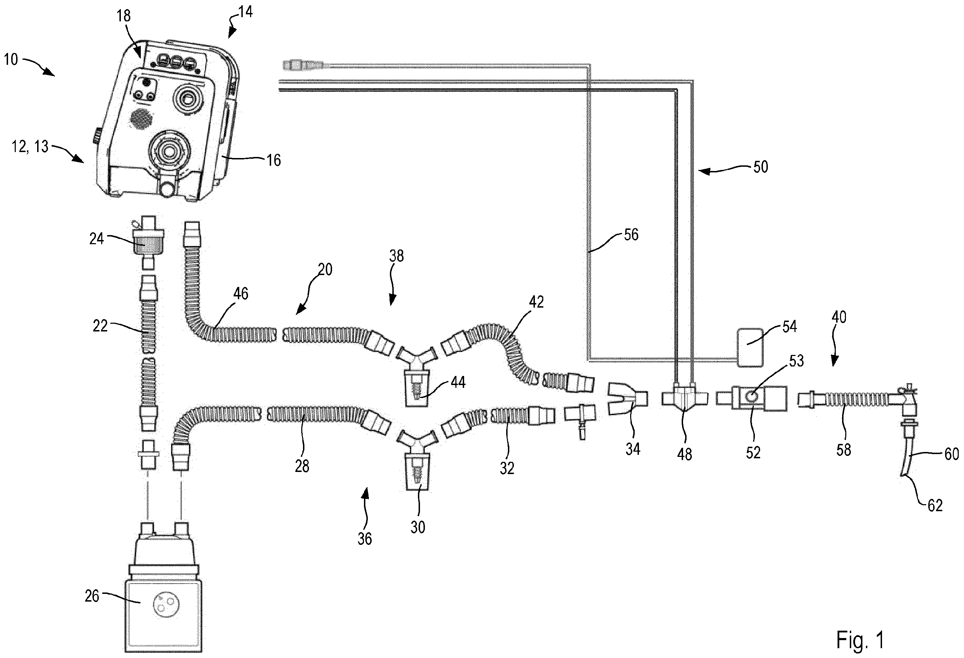

In Figur 1 ist eine erfindungsgemäße Ausführungsform einer Beatmungsvorrichtung allgemein mit 10 bezeichnet. Die Beatmungsvorrichtung 10 umfasst eine Beatmungsgasquelle 12 in Gestalt eines Gebläses sowie eine Steuervorrichtung 14 zur Einstellung von Betriebsparametern der Beatmungsgasquelle 12. Die Beatmungsgasquelle 12 und die Steuervorrichtung 14 sind im selben Gehäuse 16 aufgenommen. In diesem Gehäuse befinden sich auch an sich bekannte Ventile, wie ein Inspirationsventil und ein Exspirationsventil. Diese sind in Figur 1 jedoch nicht eigens dargestellt.

Die Steuervorrichtung 14 der Beatmungsvorrichtung 10 weist eine Eingabe/Ausgabeeinrichtung 18 auf, welche zahlreiche Schalter, wie Tastschalter und Drehschalter umfasst, um erforderlichenfalls Daten in die Steuervorrichtung 14 eingeben zu können. Das Gebläse der Beatmungsgasquelle 12 kann in seiner Förderleistung durch die Steuervorrichtung verändert werden, um die Menge an Beatmungsgas, das von der Beatmungsgasquelle pro Zeiteinheit gefördert wird, zu verändern. Die Beatmungsgasquelle 12 ist daher im vorliegenden Ausführungsbeispiel auch eine Druckveränderungsvorrichtung 13 der Beatmungsvorrichtung.

An die Beatmungsgasquelle 12 ist eine Beatmungsleitungsanordnung 20 angeschlossen, welche im vorliegenden Beispiel fünf flexible Schläuche umfasst. Ein erster inspiratorischer Beatmungsschlauch 22 verläuft von einem zwischen der Beatmungsgasquelle 12 und ihm selbst angeordneten Filter 24 zu einer Konditionierungsvorrichtung 26, wo das von der Beatmungsgasquelle 12 gelieferte Beatmungsgas auf einen vorgegebenen Feuchtegrad befeuchtet und gegebenenfalls mit Aerosol-Medikamenten versehen wird. Der Filter 24 filtert und reinigt die vom Gebläse als der Beatmungsgasquelle 12 gelieferte Umgebungsluft.

Ein zweiter inspiratorischer Beatmungsschlauch 28 führt von der Konditionierungsvorrichtung 26 zu einer inspiratorischen Wasserfalle 30. Ein dritter inspiratorischer Beatmungsschlauch 32 führt von der Wasserfalle 30 zu einem Y-Verbinder 34, welcher die distale Inspirationsleitung 36 und die distale Exspirationsleitung 38 zu einer kombinierten proximalen inspiratorisch-exspiratorischen Beatmungsleitung 40 verbindet.

Vom Y-Verbinder 34 zurück zum Gehäuse 16 verläuft ein erster exspiratorischer Beatmungsschlauch 42 zu einer exspiratorischen Wasserfalle 44 und von dort ein zweiter exspiratorischer Beatmungsschlauch 46 zum Gehäuse 16, wo das exspiratorische Beatmungsgas über ein nicht dargestelltes Exspirationsventil in die Umgebung abgelassen wird.

Auf der patientennahen kombinierten inspiratorisch-exspiratorischen Seite des Y-Verbinders 34 folgt unmittelbar auf den Y-Verbinder 34 ein Durchflusssensor 48, hier: ein Differenzdruck-Durchflusssensor 48, welcher den inspiratorischen und exspiratorischen Fluss an Beatmungsgas zum Patienten hin und vom Patienten weg erfasst. Eine Leitungsanordnung 50 überträgt den beiderseits eines Strömungshindernisses im Durchflusssensor 48 herrschenden Gasdruck an die Steuervorrichtung 14, die aus den übertragenen Gasdrücken und insbesondere aus der Differenz der Gasdrücke die Menge an pro Zeiteinheit strömendem inspiratorischem und exspiratorischem Beatmungsgas errechnet.

In Richtung vom Y-Verbinder 34 weg folgt zum Patienten hin auf den Durchflusssensor 48 eine Messküvette 52 zur nicht-dispersiven Infrarot-Erfassung eines vorbestimmten Gasanteils im Beatmungsgas. Dieser Gasanteil ist im vorliegenden Beispiel der Anteil an CO₂ im Beatmungsgas. Dabei ist sowohl der CO₂-Anteil im inspiratorischen Beatmungsgas wie auch im exspiratorischen Beatmungsgas von Interesse, da die Änderung des CO₂-Anteils zwischen Inspiration und Exploration ein Maß für die Stoffwechselfähigkeit der Patientenlunge ist. Zu erkennen ist in Figur 1 eines der seitlichen Fenster 53, durch welche Infrarotlicht in die Messküvette 52 eingestrahlt werden kann bzw. aus dieser ausstrahlen kann, je nach Orientierung eines mit der Messküvette lösbar gekoppelten Mehrkanal-Infrarot-Gassensors 54.

Der Infrarot-Gassensor 54 ist an die Messküvette 52 derart ankoppelbar, dass der Infrarot-Gassensor 54 die Messküvette 52 mit Infrarotlicht durchleuchten kann. Aus der Intensität des Infrarotlichts, genauer aus dessen spektraler Intensität kann in an sich bekannter Weise auf die Menge bzw. den Anteil eines vorbestimmten Gases in dem die Messküvette 52 durchströmenden Messgas geschlossen werden. Das vorbestimmte Gas, hier: CO₂, absorbiert Infrarotlicht einer definierten Wellenlänge. Die Intensität des Infrarotlichts in dieser Wellenlänge hängt nach Durchgang im Wesentlichen von der Absorption des Infrarotlichts dieser Wellenlänge durch das vorbestimmte Gas ab. Ein Vergleich der Intensität des Infrarotlichts der definierten Wellenlänge mit einer Wellenlänge des Infrarotlichts, die zu keinem Absorptionsspektrum eines zu erwartenden Gasanteils im Messgas gehört, liefert eine Information über den Anteil des vorbestimmten Gases am Messgas. Der IR-Gassensor 54 ist daher über eine Datenleitung 56 mit der Steuervorrichtung 14 der Beatmungsvorrichtung 10 verbunden und überträgt über die Datenleitung 56 die beschriebenen Intensitätsinformationen an die Steuervorrichtung 14.

Auf die Messküvette 52 folgt in Richtung zum Patienten hin ein weiteres Schlauchstück 58, an welchen ein Endotrachealtubus 60 als Beatmungsschnittstelle zum Patienten angeordnet ist. Eine proximale Öffnung 62 des Endotrachealtubus 60 ist sowohl Beatmungsgas-Auslassöffnung, durch welche inspiratorisches Beatmungsgas durch den Endotrachealtubus 60 in den Patienten eingeleitet wird, als auch Beatmungsgas-Einlassöffnung, durch welche exspiratorisches Beatmungsgas aus dem Patienten zurück in den Endotrachealtubus 60 geleitet wird.

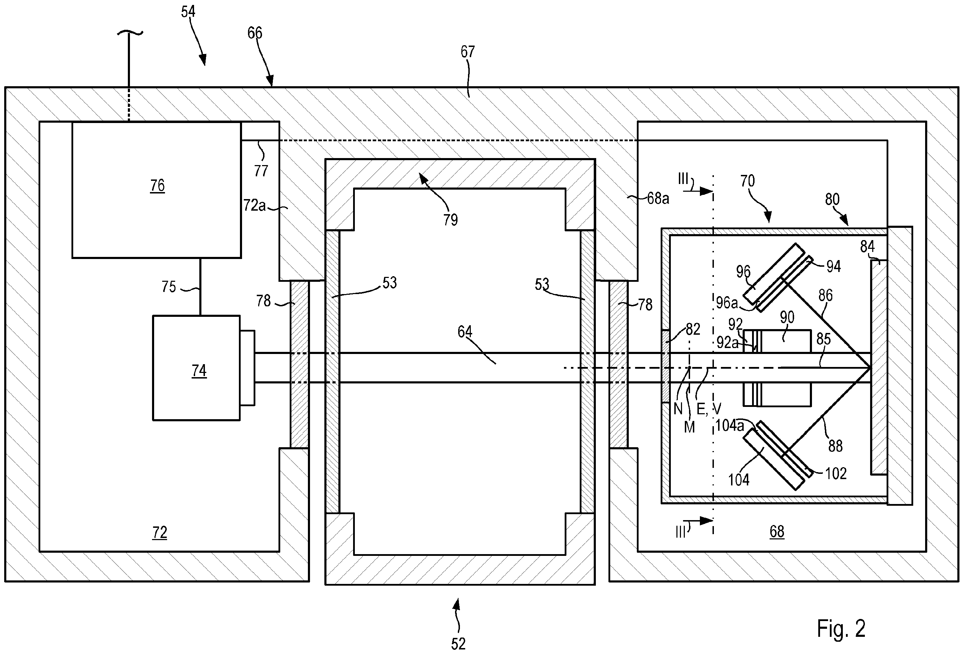

In Figur 2 sind die Messküvette 52 grobschematisch im Querschnitt und der darin angekoppelte Mehrkanal-IR-Gassensor 54 grobschematisch im Längsschnitt dargestellt. Die Messküvette 52 in Figur 2 wird orthogonal zur Zeichenebene von Figur 2 mit Beatmungsgas durchströmt. Ein Infrarotstrahl 64 des Gassensors 54 verläuft parallel zur bzw. in der Zeichenebene von Figur 2.

Der Gassensor 54 umfasst ein Sensorgehäuse 66, in dessen erstem Kompartiment 68 eine weiter unten näher erläuterte Sensorik 70 angeordnet ist, und umfasst ein zweites Kompartiment 72, in welchem eine Infrarot-Strahlenquelle 74 angeordnet ist. Lediglich beispielhaft ist im zweiten Kompartiment 72 eine bordeigene Sensor-Steuervorrichtung 76 angeordnet, welche über Leitungen 75 und 77 mit der Infrarot-Strahlenquelle 74 und der Sensorik 70 signalübertragend kommuniziert und welche über die Datenleitung 56 signalübertragend mit der Steuervorrichtung 14 kommuniziert. Im vorliegenden Beispiel kann die Steuervorrichtung 14 der Beatmungsvorrichtung 10 als übergeordnete Steuervorrichtung des IR-Gassensors 54 agieren und Erfassungswerte von der Sensor-Steuervorrichtung 76 anfordern, die daraufhin die Infrarot-Strahlenquelle 74 entsprechend zum Betrieb ansteuert und die von der Sensorik 70 erfassten Erfassungssignale an die Steuervorrichtung 14 zur Auswertung durch diese übertragt. Die Steuervorrichtung 14 ist somit eine Auswertevorrichtung des IR-Gassensors 54.

Die beiden Kompartimente 68 und 72 sind durch eine Gehäusebrücke 67 überbrückt. Die Gehäusebrücke 67 und die daran anschließenden Seitenwände 68a und 72a der beiden Kompartimente 68 und 72 bilden eine Klemm-Aufnahmeformation 79, in welche die Messküvette 52 eingeführt und lösbar klemmend verankert werden kann. Durch bloßes manuelles Überwinden der Klemmkraft können die Messküvette 52 und das Gehäuse 66 des Gassensors 54 wieder voneinander getrennt werden. Zusätzlich oder alternativ können Verrastungsmittel zur Verrastung von Gassensor und Messküvette 52 aneinander vorgesehen sein.

Jedes der Kompartiment der 68 und 72 weist je ein Infrarot durchlässiges Fenster 78 auf, welches von dem von der Infrarot-Strahlenquelle 74 ausgestrahlten Infrarotstrahl 64 durchstrahlt wird. Da der Infrarotstrahl 64 die Messküvette 52 vollständig durchqueren muss, weist die Messküvette 52 beiderseits des durch sie bestimmten Strömungsdurchgangs je ein Fenster 53 auf, welches für Infrarotlicht durchlässig ist und welches von dem Infrarotstrahl 64 ebenso durchquert wird. Die Messküvette 52 ist in dem im IR-Gassensor 54 aufgenommenen Abschnitt bevorzugt spiegelsymmetrisch bezüglich einer zum Infrarotstrahl 64 orthogonalen Spiegelsymmetrieebene ausgebildet, da es auf die Richtung der Durchstrahlung der Messküvette 52 durch den Infrarotstrahl 64 nicht ankommt. Der IR-Gassensor 54 kann daher abweichend von der Darstellung der Figur 2 auch um 180° um eine zur Zeichenebene der Figur 2 und zum Infrarotstrahl 64 orthogonale Achse gedreht mit der Messküvette 52 gekoppelt sein.

Die Sensorik 70 weist ein eigenes Sensorik-Gehäuse 80 auf. Das Sensorik-Gehäuse 80 umfasst ein Fenster 82, durch welches der Infrarotstrahl 64 längs einer Einstrahlachse E in das Sensorik-Gehäuse 80 einstrahlen kann.

Nach dem Durchgang durch das Fenster 82 trifft der einstrahlende Infrarotstrahl 64 auf eine Strahlenteileranordnung 84, welche eine Vielzahl von nicht dargestellten pyramidonalen Reflexionskörpern aufweist und welche einen auf sie einstrahlenden Anteil des Infrarotstrahls 64 unter bezüglich der Einstrahlachse E betragsgleichen Winkeln in vier Infrarot-Teilstrahlen teilt. Von diesen vier Infrarot-Teilstrahlen liegen je zwei Infrarot-Teilstrahlen mit entgegengesetzten Teilstrahlen-Verlaufskomponenten in derselben Teilstrahlenebene, wobei die beiden Teilstrahlenebenen zueinander orthogonal sind und im dargestellten Beispiel die Einstrahlachse E als gemeinsame Schnittachse enthalten.

Parallel zur Zeichenebene von Figur 2 verlaufen der zweite Infrarot-Teilstrahl 86 und der vierte Infrarot-Teilstrahl 88. Diese sind in Figur 2 gezeigt. Sie schließen mit der Einstrahlachse E jeweils einen Winkel mit einem Betrag von etwa 45° ein.

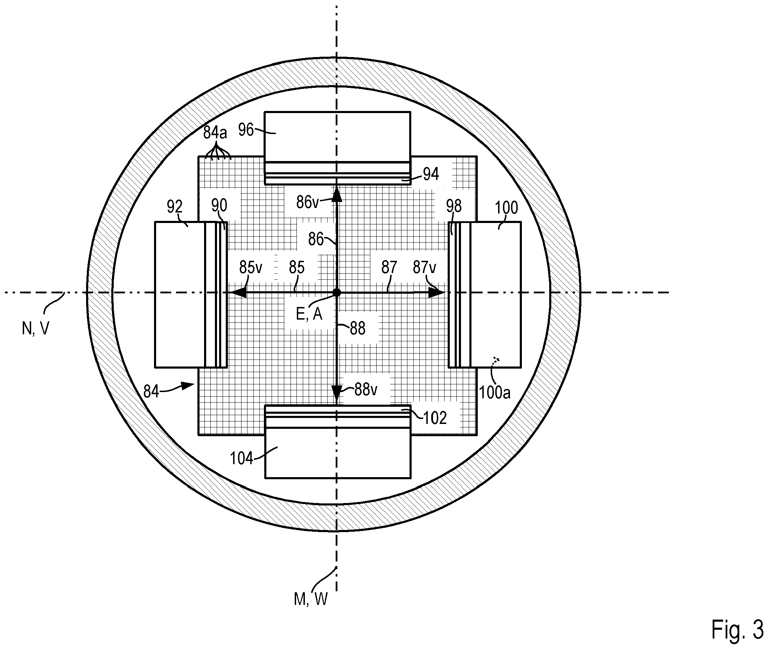

Ein erster Infrarot-Teilstrahl 85 verläuft ebenfalls unter einem Winkel von etwa 45° zur Einstrahlachse E vom Betrachter der Figur 2 weg zu dem ersten Bandfilter 90 und zu dem hinter diesem gelegenen ersten Infrarot-Nutzsignal-Sensor 92. Der erste Infrarot-Teilstrahl 85 erreicht den ersten Infrarot-Nutzsignal-Sensor 92 also erst nach Durchgang durch den ersten Bandfilter 90. Ein vierter Infrarot-Teilstrahl 87 ist in Figur 2 nicht dargestellt, da er vollständig vor der Zeichenebene der Figur 2 gelegen ist. Er ist in Figur 3 dargestellt.

Ein zweiter Bandfilter 94 befindet sich im Strahlengang des zweiten Infrarot-Teilstrahls 86 vor dem ersten Infrarot-Referenzsignal-Sensor 96.

Ein in Figur 2 nicht dargestellter, weil vor der Zeichenebene von Figur 2 gelegener dritter Bandfilter 98 befindet sich im Strahlengang des dritten Infrarot-Teilstrahls 87 vor dem zweiten Infrarot-Nutzsignal-Sensor 100 (siehe Figur 3).

Schließlich befindet sich ein vierter Bandfilter 102 im Strahlengang des vierten Infrarot-Teilstrahls 88 vor einem zweiten Infrarot-Referenzsignal-Sensor 104.

Die Pfeilspitzen in Figur 3 geben die paarweise verschiedenen Verlaufsrichtungen 85v, 86v, 87v und 88v der ersten bis vierten Infrarot-Teilstrahlen 85, 86, 87 und 88 bzw ihrer Strahlengänge an.

Der erste und der dritte Bandfilter 90 bzw. 98 weisen ein Transmissionsmaximum im Bereich der Absorptionswellenlängen von CO₂ auf, etwa in einem Bereich zwischen 4,25 µm und 4,28 µm. Ihre Bandbreiten liegen im Bereich von 170 bis 180 nm.

Der zweite und der vierte Bandfilter 94 bzw. 102 weisen ein Transmissionsmaximum im Bereich außerhalb der Absorptionswellenlängen von CO₂ auf, etwa im Bereich von 3,90 µm bis 4,0 µm, oder/und im Bereich von 4,40 µm bis 4,5 µm. Ihre Bandbreiten liegen im Bereich von 60 bis 90 nm.

Die IR-Sensoren 92, 96, 100 und 104 sind bevorzugt baugleich. Es handelt sich um pyroelektrische IR-Sensoren, die orthogonal zu ihrer jeweiligen Sensor-Erfassungsfläche 92a, 96a und 104a piezoelektrisch sensitiv sind. Dies gilt auch für den zweiten IR-Nutzsignal-Sensor 98, dessen Sensor-Erfassungsfläche 100a jedoch nur in Figur 3 dargestellt ist. Die Sensor-Erfassungsflächen 92a, 96a, 100a und 104a der IR-Sensoren 92, 96, 100 und 104 sind eben.

Die Sensor-Erfassungsflächen 96a und 104a des ersten und des zweiten IR-Referenzsignal-Sensors 96 bzw. 104 sind zueinander um eine zur Zeichenebene der Figur 2 orthogonale Neigeachse N um 90° geneigt. Die Sensor-Erfassungsflächen 96a und 104a weisen somit sowohl aufeinander zu als auch zur Strahlenteileranordnung 84 hin. Der erste und der zweite IR-Referenzsignal-Sensor 96 bzw. 104 sind bezüglich einer zur Zeichenebene der Figur 2 orthogonalen, die Einstrahlachse E und die Neigeachse N enthaltenden Referenzsignal-Sensor-Symmetrieebene V spiegelsymmetrisch orientiert. Dasselbe gilt für den ersten und den zweiten IR-Nutzsignal-Sensor 92 bzw. 98, jedoch ist deren Nutzsignal-Sensor-Symmetrieebene W parallel zur Zeichenebene von Figur 2.Sie enthält jedoch ebenfalls die Einstrahlachse E. Die Neigeachse M, um welche die Sensor-Erfassungsfläche 92a des ersten IR-Nutzsignal-Sensors und die Sensor-Erfassungsfläche 100a des zweiten IR-Nutzsignal-Sensors 100 zueinander um etwa 90° geneigt sind, liegt mit der Neigeachse N in einer gemeinsamen zur Einstrahlachse E orthogonalen Ebene und schließt mit der Neigeachse N einen rechten Winkel ein.

Andersherum ausgedrückt spannen die Neigeachse N und die Einstrahlachse E im Ausführungsbeispiel die Symmetrieebene V auf und spannen die Neigeachse M und die Einstrahlachse E die Symmetrieebene W auf, wie dies in Figur 3 zu erkennen ist.

Die symmetrisch bezüglich der Symmetrieebene V orientierten IR-Resonanzsignal-Sensoren 96 und 104 und ebenso deren zugeordnete Bandfilter 94 bzw. 102 sind bezüglich der Symmetrieebene W spiegelsymmetrisch ausgebildet. Ebenso gilt, dass die symmetrisch bezüglich der Symmetrieebene W orientierten IR-Nutzsignal-Sensoren 92 und 100 ebenso wie ihre zugeordneten Bandfilter 90 bzw. 98 spiegelsymmetrisch zur Symmetrieebene V angeordnet sind.

Wie in Figur 3 gezeigt ist sind die IR-Sensoren 92, 96, 100 und 104 um eine mit der Einstrahlachse E zusammenfallende Anordnungsachse A herum alternierend unter gleichem Winkelabstand von 90° angeordnet. Auf einen IR-Nutzsignal-Sensor folgt in einer Umlaufrichtung um die Anordnungsachse A stets ein IR-Referenzsignal-Sensor und umgekehrt. Die in Figur 2 nicht sichtbare Sensor-Erfassungsfläche 100a ist in Figur 3 bezeichnet.

Die kleinen Quadrate in der Strahlenteileranordnung 84 symbolisieren die in Zeilen und Spalten angeordneten pyramidonalen Reflexionskörper 84a, deren Pyramidenachse zueinander und zur Einstrahlachse E parallel sind.

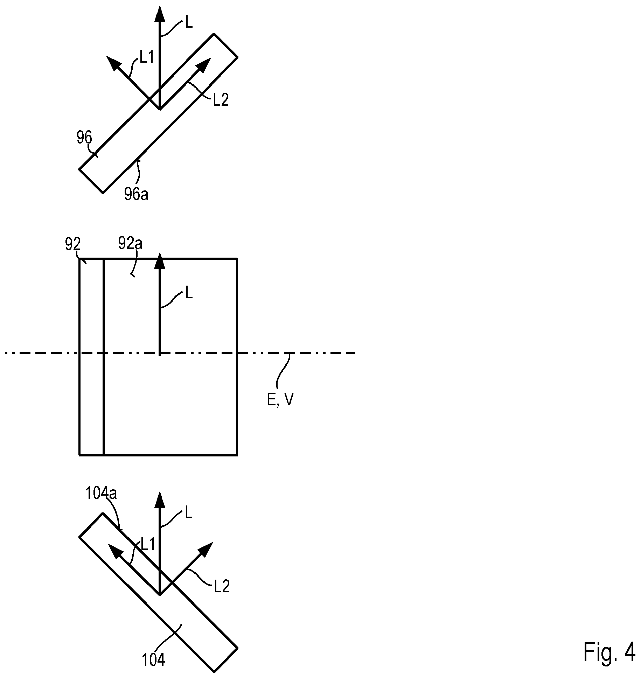

In Figur 4 ist erläutert, wie sich eine mechanische Belastung auf die Sensorik 70 auswirkt. Es sei eine mechanische Stoßbelastung L angenommen, welche parallel zur Zeichenebene der Figuren 3 und 4 und damit parallel zur Symmetrieebene W sowie orthogonal zur Einstrahlachse E und damit zur Anordnungsachse A wirkt. Die mechanische Stoßbelastung L ist an den in Figur 2 gezeigten und in Figur 4 wiederholt dargestellten IR-Sensoren 92, 96 und 104 gezeigt. Für den in den Figuren 2 und 4 nicht dargestellten IR-Nutzsignal-Sensor 100 gilt aufgrund der symmetrischen Orientierung der IR-Nutzsignal-Sensoren 92 und 100 das zum IR-Nutzsignal-Sensor 92 Gesagte entsprechend.

Die pyroelektrischen IR-Sensoren 92, 96, 100 und 104 weisen orthogonal zu ihren Sensor-Erfassungsflächen 92a, 96a, 100a und 104a eine piezoelektrische Sensitivität auf, d. h. eine mechanische Belastung mit einer Komponente orthogonal zur Sensor-Erfassungsfläche löst in dem betroffenen IR-Sensor ein elektrisches Signal aus, dessen Polarität abhängig von der Wirkungsrichtung der mechanischen Belastung orthogonal zur Sensor-Erfassungsfläche ist.

Die IR-Nutzsignal-Sensoren 92 und 100 sind mit ihren Sensor-Erfassungsflächen 92a und 100a parallel zur mechanischen Stoßbelastung L orientiert, sodass die mechanische Stoßbelastung L keine Komponente orthogonal zu deren Sensor-Erfassungsflächen 92a und 100a aufweist. Die mechanische Stoßbelastung L stört folglich das IR-Erfassungssignal der IR-Nutzsignal-Sensoren 92 und 100 nicht.

An den IR-Referenzsignal-Sensoren 96 und 104 lässt sich die mechanische Stoßbelastung L in zueinander orthogonale Komponenten L1 und L2 zerlegen, von welchen Komponente L1 orthogonal zur Sensor-Erfassungsfläche 96a des ersten IR-Referenzsignal-Sensors 96 orientiert ist und von welchen die Komponente L2 orthogonal zur Sensor-Erfassungsfläche 104a des zweiten IR-Referenzsignal-Sensors 104 orientiert ist. Wegen der betragsgleichen, aber entgegengesetzt gerichteten Winkelorientierung der Sensor-Erfassungsflächen 96a und 104a relativ zur Anordnungsachse A bzw. zur Einstrahlachse E sind die Beträge der zueinander orthogonalen Komponenten L1 und L2 gleich groß, jedoch ist die Komponente L1 bezüglich der Sensor-Erfassungsfläche 96a entgegengesetzt gerichtet, verglichen mit der Komponente L2 und ihrer Orientierung zur Sensor-Erfassungsfläche 104a. Die mechanische Stoßbelastung L stört daher in jedem der IR-Referenzsignal-Sensoren 96 und 104 das eigentliche infrarotinduzierte Erfassungssignal und überlagert diesem ein durch die mechanische Stoßbelastung L induziertes Störsignal. Die beiden Störsignale der IR-Referenzsignal-Sensoren 96 und 104 sind jedoch synchron und entgegengesetzt gerichtet, sodass sie durch entsprechende Signalverarbeitung, etwa durch Signaladdition der Erfassungssignale der IR-Referenzsignal-Sensoren 96 und 104 ausgelöscht werden können. Das Signal der Referenzsignal-Sensoren 96 und 104 ist dann ebenso störungsfrei wie das der Nutzsignal-Sensoren 92 und 100.

Da die Referenzsignal-Sensor-Symmetrieebene V und die Nutzsignal-Sensor-Symmetrieebene W sich in der Anordnungsachse A schneiden und folglich beide Ebenen V und W die Anordnungsachse A enthalten, gilt für jede zur Anordnungsachse A orthogonale mechanische Belastung, dass deren Auswirkungen auf das Signal in bestimmungsgleichen IR-Sensoren 92 und 100 einerseits sowie 96 und 104 andererseits durch entsprechende Signalbearbeitung der bestimmungsgleichen Sensoren ausgelöscht oder sehr stark reduziert werden kann.

Längs der Anordnungsachse A verlaufende mechanische Belastungen oder Belastungskomponenten lassen sich so zwar nicht innerhalb von bestimmungsgleichen IR-Sensoren auslöschen oder stark reduzieren. Jedoch führen bei der Anordnung der Ausführungsform, bei welcher alle IR-Sensoren 92, 96, 100 und 104 relativ zur Anordnungsachse A mit betragsgleichen Winkeln orientiert sind, zu einem identischen Störeinfluss der Belastung auf alle IR-Sensoren, sodass der Einfluss der mechanischen Belastung auf die Richtigkeit des aus den Signalen des Gassensors 54 erhaltenen Erfassungsergebnisses hinsichtlich des CO₂-Anteils am Messgas erheblich reduziert werden kann, da die längs der Anordnungsachse A wirkende mechanische Belastung das aus den Signalen der IR-Nutzsignal-Sensoren 92 und 100 erhaltene Nutzsignal und das aus den Signalen der IR-Referenzsignal-Sensoren 96 und 104 erhaltene Referenzsignal im Wesentlichen synchron, betragsgleich und richtungsgleich beeinflusst. Der für die Ermittlung der Absorption von Infrarotlicht eigentlich wichtige Unterschied zwischen den Signalpegeln der IR-Nutzsignal-Sensoren 92 und 100 einerseits und den Signalpegeln der IR-Referenzsignal-Sensoren 96 und 104 andererseits ändert sich nicht oder nur in vernachlässigbarem Umfang.

Da jede mechanische Belastung in eine Komponente parallel zur Anordnungsachse A und in eine Komponente orthogonal zu dieser unterteilt werden kann, gestattet die vorliegend vorgestellte Sensorik 70 die weitgehende Elimination von Störwirkungen von auf die Sensorik 70 einwirkenden mechanischen Belastungen.

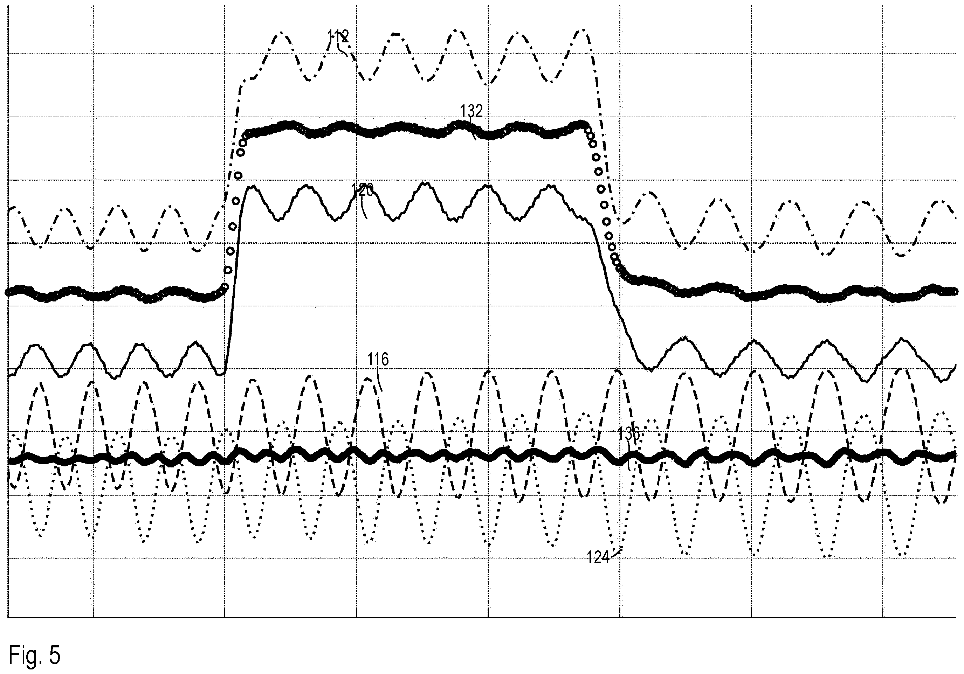

In Figur 5 sind längs der Abszisse als Zeitachse Signale der IR-Sensoren 92, 96, 100 und 104 aufgetragen. Die Ordinate von Figur 5 gibt den Pegel der jeweiligen Signale an.

Das Signal 112 stammt vom ersten IR-Nutzsignal-Sensor 92, das Signal 116 vom ersten IR-Referenzsignal-Sensor 96, das Signal 120 vom zweiten IR-Nutzsignal-Sensor 100 und das Signal 124 vom zweiten IR-Referenzsignal-Sensor 104.

Aus den Signalen 112 und 120 wird ein Nutzsignal 132 erhalten, beispielsweise durch Mittelwertbildung. Aus den Signalen 116 und 124 wird ein Referenzsignal 136, beispielsweise ebenfalls durch Mittelwertbildung, erhalten.

Obwohl beide Signale 116 und 124 der IR-Referenzsignal-Sensoren 96 und 104 jeweils durch mechanische Belastung gestört sind, was an der periodischen Schwingung des jeweiligen Signalpegels erkennbar ist, ist das daraus resultierende Referenzsignal 136 durch die mechanische Belastung kaum beeinflusst. Gleiches gilt für die Signale 112 und 120 der IR-Nutzsignal-Sensoren 92 und 100 und daraus erhaltene Nutzsignal 132 im Verhältnis dazu.

In Figur 5 haben die von den IR-Nutzsignal-Sensoren 92 und 100 stammenden Signale 112 und 120 sowie das daraus gebildete Nutzsignal 132 zunächst einen niedrigen Pegel, welche auf Absorption von Infrarotlicht des Infrarotstrahls 64 durch einen CO₂-Anteil im Messgas in der Messküvette 52 zurückzuführen ist. Hierauf folgt ein Signal mit höherem Pegel, in welchem weniger CO₂ im Messgas der Messküvette 52 vorhanden ist und deshalb weniger Infrarotlicht im Wellenlängenbereich der Bandfilter 90 und 98 absorbiert wird. Auf diese Phase mit höherem Pegel folgt wieder eine Phase mit niedrigeren Pegeln der Signale 112 und 120 sowie folglich auch des Nutzsignals 132. Die Signale der Figur 5 entstammen keiner realen Beatmungssituation, sondern sind durch einen Versuchsaufbau im Labor erhalten.

Ebenso ist die mechanische Belastung der IR-Sensoren 92, 96, 100 und 104, die zu den einzelnen schwingenden Signalen 112, 116, 120 und 124 führt durch einen Vibrationstisch induziert. Sie entspricht keiner stochastisch-zufälligen mechanischen Belastung eines realen medizinischen Einsatzes des Gassensors 54.

## Patentansprüche

1. Mehrkanal-Infrarot-Gassensor (54), umfassend
- eine Strahlenteileranordnung (84), welche dazu ausgebildet ist, einen auf die Strahlenteileranordnung (84) längs einer vorbestimmten Einstrahlachse (E) einstrahlenden Infrarotstrahl (64) in eine Mehrzahl von Infrarot-Teilstrahlen (85, 86, 87, 88) zu teilen,
- einen in einem ersten Strahlengang eines ersten Infrarot-Teilstrahls (85) angeordneten ersten Bandfilter (90) mit einer vorbestimmten ersten Bandbreite und mit einem Transmissionsmaximum bei einer vorbestimmten ersten Nutzsignal-Wellenlänge,
- einen im ersten Strahlengang des ersten Infrarot-Teilstrahls (85) hinter dem ersten Bandfilter (90) angeordneten ersten Infrarot-Nutzsignal-Sensor (92),
- einen zweiten Bandfilter (94), welcher in einem zweiten Strahlengang eines vom ersten Infrarot-Teilstrahl (58) verschiedenen zweiten Infrarot-Teilstrahls (86) angeordnet ist, wobei der zweite Bandfilter (94) eine vorbestimmte zweite Bandbreite und ein Transmissionsmaximum bei einer vorbestimmten ersten Referenzsignal-Wellenlänge aufweist, wobei die erste Referenzsignal-Wellenlänge von der ersten Nutzsignal-Wellenlänge verschieden ist,
- einen im zweiten Strahlengang des zweiten Infrarot-Teilstrahls (86) hinter dem zweiten Bandfilter (94) gelegenen ersten Infrarot-Referenzsignal-Sensor (96),
wobei die Strahlenteileranordnung (84) dazu ausgebildet ist, den einstrahlenden Infrarotstrahl (64) in wenigstens vier Infrarot-Teilstrahlen (85, 86, 87, 88) zu teilen, wobei der Mehrkanal-Infrarot-Gassensor (54) weiter umfasst:
- einen dritten Bandfilter (98), welcher in einem dritten Strahlengang eines dritten Infrarot-Teilstrahls (87) angeordnet ist, wobei der dritte Bandfilter (98) eine vorbestimmte dritte Bandbreite und ein Transmissionsmaximum bei einer vorbestimmten zweiten Nutzsignal-Wellenlänge aufweist,
- einen im dritten Strahlengang des dritten Infrarot-Teilstrahls (87) hinter dem dritten Bandfilter (98) gelegenen zweiten Infrarot-Nutzsignal-Sensor (100),
- einen vierten Bandfilter (102), welcher in einem vierten Strahlengang eines vierten Infrarot-Teilstrahls (88) angeordnet ist, wobei der vierte Bandfilter (102) eine vorbestimmte vierte Bandbreite und ein Transmissionsmaximum bei einer vorbestimmten zweiten Referenzsignal-Wellenlänge aufweist,
- einen im vierten Strahlengang des vierten Infrarot-Teilstrahls (88) hinter dem vierten Bandfilter (102) gelegenen zweiten Infrarot-Referenzsignal-Sensor (104),
wobei die Verlaufsrichtungen (85v, 86, 87v, 88v) des ersten, des zweiten, des dritten und des vierten Strahlengangs paarweise voneinander verschieden sind, wobei jede Wellenlänge aus der ersten und der zweiten Referenzsignal-Wellenlänge von jeder Wellenlänge aus der ersten und der zweiten Nutzsignal-Wellenlänge verschieden ist, wobei der erste und der zweite Infrarot-Nutzsignal-Sensor (92, 100) derart angeordnet sind, dass ihre jeweiligen Nutzsignal-Sensor-Erfassungsflächen (92a, 100a) bezüglich einer zwischen den Nutzsignal-Sensor-Erfassungsflächen (92a, 100a) gelegenen Nutzsignal-Sensor-Symmetrieebene (W) symmetrisch orientiert sind, und wobei der erste und der zweite Infrarot-Referenzsignal-Sensor (96, 104) derart angeordnet sind, dass ihre jeweiligen Referenzsignal-Sensor-Erfassungsflächen (96a, 104a) bezüglich einer zwischen den Referenzsignal-Sensor-Erfassungsflächen (96a, 104a) gelegenen Referenzsignal-Sensor-Symmetrieebene (V) symmetrisch orientiert sind, wobei keine Nutzsignal-Sensor-Erfassungsfläche (92a, 100a) orthogonal zur Nutzsignal-Sensor-Symmetrieebene (W) orientiert ist und wobei keine Referenzsignal-Sensor-Erfassungsfläche (96a, 1004a) orthogonal zur Referenzsignal-Sensor-Symmetrieebene (V) orientiert ist,
**dadurch gekennzeichnet, dass** der erste Infrarot-Nutzsignal-Sensor (92) und der zweite Infrarot-Nutzsignal-Sensor (100) derart angeordnet sind, dass ihre jeweiligen Nutzsignal-Sensor-Erfassungsflächen (92a, 100a) um eine virtuelle Nutzsignal-Neigeachse (M) aufeinander zu geneigt sind, und dass der erste Infrarot-Referenzsignal-Sensor (96) und der zweite Infrarot-Referenzsignal-Sensor (104) derart angeordnet sind, dass ihre jeweiligen Referenzsignal-Sensor-Erfassungsflächen (96a, 104a) um eine von der Nutzsignal-Neigeachse (M) verschiedene virtuelle Referenzsignal-Neigeachse (N) aufeinander zu geneigt sind.

2. Mehrkanal-Infrarot-Gassensor (54) nach Anspruch 1,
**dadurch gekennzeichnet, dass** Nutzsignal-Sensor-Symmetrieebene (W) und die Referenzsignal-Sensor-Symmetrieebene (V) voneinander verschieden sind.

3. Mehrkanal-Infrarot-Gassensor (54) nach Anspruch 1 oder 2,
**dadurch gekennzeichnet, dass** die Strahlenteileranordnung (84) eine Reflektor-Strahlenteileranordnung ist und dass sich alle Infrarot-Nutzsignal-Sensoren (92, 100) sowie alle Infrarot-Referenzsignal-Sensoren (96, 104) auf einer Einstrahlseite der Strahlenteileranordnung (84) befinden, auf welcher ein in den Gassensor (54) eintretender Infrarotstrahl (64) auf die Strahlenteileranordnung (84) einstrahlt.

4. Mehrkanal-Infrarot-Gassensor (54) nach Anspruch 3,
**dadurch gekennzeichnet, dass** die Infrarot-Nutzsignal-Sensoren (92, 100) und die Infrarot-Referenzsignal-Sensoren (96, 104) alternierend in Umfangsrichtung um eine virtuelle Anordnungsachse (A) angeordnet sind.

5. Mehrkanal-Infrarot-Gassensor (54) nach Anspruch 4,
**dadurch gekennzeichnet, dass** der erste und der zweite Infrarot-Nutzsignal-Sensor (92, 100) einander bezüglich der virtuellen Anordnungsachse (A) diametral gegenüberliegen oder/und dass der erste und der zweite Infrarot-Referenzsignal-Sensor (96, 104) einander bezüglich der virtuellen Anordnungsachse (A) diametral gegenüberliegen.

6. Mehrkanal-Infrarot-Gassensor (54) nach Anspruch 4 oder 5,
**dadurch gekennzeichnet, dass** die virtuelle Anordnungsachse (A) die Einstrahlachse (E) ist.

7. Mehrkanal-Infrarot-Gassensor (54) nach einem der Ansprüche 4 bis 6,
**dadurch gekennzeichnet, dass** die Nutzsignal-Sensor-Symmetrieebene (W) oder/und die Referenzsignal- Sensor-Symmetrieebene (V) die Anordnungsachse (A) enthält bzw. enthalten oder parallel zur Anordnungsachse (A) verläuft bzw. verlaufen.

8. Mehrkanal-Infrarot-Gassensor (54) nach einem der vorhergehenden Ansprüche,
**dadurch gekennzeichnet, dass** die Sensor-Erfassungsfläche (92a, 100a) jedes Infrarot-Nutzsignal-Sensors (92, 100) und die Sensor-Erfassungsfläche (96a, 104a) jedes Infrarot-Referenzsignal-Sensors (96, 104) zur Strahlenteileranordnung (84) hin geneigt ist.

9. Mehrkanal-Infrarot-Gassensor (54) nach einem der vorhergehenden Ansprüche,
**dadurch gekennzeichnet, dass** die erste oder/und die zweite Nutzsignal-Wellenlänge betragsmäßig zwischen der ersten und der zweiten Referenzsignal-Wellenlänge gelegen ist bzw. sind.

10. Mehrkanal-Infrarot-Gassensor (54) nach einem der vorhergehenden Ansprüche,
**dadurch gekennzeichnet, dass** die erste und die zweite Nutzsignal-Wellenlänge sich betragsmäßig um nicht mehr als ein Drittel der betragsmäßig kleineren Bandbreite aus erster und dritter Bandbreite unterscheiden.

11. Mehrkanal-Infrarot-Gassensor (54) nach einem der vorhergehenden Ansprüche,
**dadurch gekennzeichnet, dass** jede Bandbreite aus zweiter und vierter Bandbreite betragsmäßig kleiner ist als jede Bandbreite aus erster und dritter Bandbreite.

12. Mehrkanal-Infrarot-Gassensor (54) nach einem der vorhergehenden Ansprüche,
**dadurch gekennzeichnet, dass** der Gassensor (54) eine Auswertevorrichtung (14) umfasst, welche aus den Signalen des ersten und des zweiten Infrarot-Referenzsignal-Sensors (96, 104) eine Referenzinformation (136) ermittelt, welche aus den Signalen des ersten und des zweiten Infrarot-Nutzsignal-Sensors (92, 100) eine Nutzinformation (132) ermittelt, und welche aus einem Vergleich von Referenzinformation (136) und Nutzinformation (132) eine Information über einen Anteil eines durch die erste oder/und die zweite Nutzsignal-Wellenlänge identifizierten Gases an einem mit dem einstrahlenden Infrarotstrahl (64) bestrahlten Messgas ausgibt.

13. Mehrkanal-Infrarot-Gassensor (54) nach einem der vorhergehenden Ansprüche,
**dadurch gekennzeichnet, dass** der Gassensor (54) ein Sensorgehäuse (66) aufweist mit einem ersten Kompartiment (68), in welchem die Strahlenteileranordnung (84), die Infrarot-Nutzsignal-Sensoren (92, 100) und die Infrarot-Referenzsignal-Sensoren (96) angeordnet sind, und mit einem räumlich vom ersten Kompartiment (68) entfernt gelegenen zweiten Kompartiment (72), in welchem eine Infrarot-Strahlenquelle (74) angeordnet ist, wobei zwischen dem ersten und dem zweiten Kompartiment (68, 72) eine Aufnahmeformation (79) zur Aufnahme einer Messküvette (52) zwischen dem ersten und dem zweiten Kompartiment (68, 72) angeordnet ist.

14. Beatmungsvorrichtung (10) zur wenigstens unterstützend künstlichen Beatmung eines lebenden Patienten, umfassend:
- eine Beatmungsgasquelle (12),
- eine Beatmungsleitungsanordnung (20), um inspiratorisches Beatmungsgas von der Beatmungsgasquelle (12) zu einer patientenseitigen proximalen Beatmungsgas-Auslassöffnung (62) und um exspiratorisches Beatmungsgas von einer proximalen Beatmungsgas-Einlassöffnung (62) weg zu leiten,
- eine Druckveränderungsvorrichtung (13) zur Veränderung des Drucks des Beatmungsgases in der Beatmungsleitungsanordnung (20),
- eine Steuervorrichtung (14) zum Betrieb der Beatmungsgasquelle (12) oder/und der Druckveränderungsvorrichtung (13) sowie
- einen Mehrkanal-Infrarot-Gassensor (54) nach einem der vorhergehenden Ansprüche zur Erfassung wenigstens eines Gasbestandteils im inspiratorischen oder/und exspiratorischen Beatmungsgas.

15. Beatmungsvorrichtung (10) nach Anspruch 14,
**dadurch gekennzeichnet, dass** der Mehrkanal-Infrarot-Gassensor (54) ein Gassensor (54) nach Anspruch 11 ist, wobei die Auswertevorrichtung (14) Teil der Steuervorrichtung (14) der Beatmungsvorrichtung (10) ist.

## Claims

1. A multi-channel infrared gas sensor (54) comprising
- A beam splitter assembly (84) adapted to split an infrared beam (64) incident on the beam splitter assembly (84) along a predetermined irradiation axis (E) into a plurality of infrared partial beam (85, 86, 87, 88),
- A first band filter (90) arranged in a first beam path of a first infrared partial beam (85) and having a predetermined first bandwidth and having a transmission maximum at a predetermined first useful signal wavelength,
- A first infrared useful signal sensor (92) arranged in the first beam path of the first infrared partial beam (85) behind the first band filter (90),
- A second band filter (94) arranged in a second beam path of a second infrared partial beam (86) different from the first infrared partial beam (58), the second band filter (94) having a predetermined second bandwidth and a transmission maximum at a predetermined first reference signal wavelength, the first reference signal wavelength being different from the first useful signal wavelength,
- A first infrared reference signal sensor (96) located in the second beam path of the second infrared partial beam (86) behind the second band filter (94),
wherein the beam splitter arrangement (84) is adapted to split the incoming infrared beam (64) into at least four infrared partial beams (85, 86, 87, 88), the multi-channel infrared gas sensor (54) further comprising:
- A third band filter (98) which is arranged in a third beam path of a third infrared partial beam (87), the third band filter (98) having a predetermined third bandwidth and a transmission maximum at a predetermined second useful signal wavelength,
- A second infrared useful signal sensor (100) located in the third beam path of the third infrared partial beam (87) behind the third band filter (98)
- A fourth band filter (102) arranged in a fourth beam path of a fourth infrared partial beam (88), the fourth band filter (102) having a predetermined fourth bandwidth and a transmission maximum at a predetermined second reference signal wavelength,
- a second infrared reference signal sensor (104) arranged in the fourth beam path of the fourth infrared partial beam (88) behind of the fourth band filter (102),
wherein the directions (85v, 86, 87v, 88v) of the first, second, third and fourth beam paths are different from each other in pairs, each of the first and second reference signal wavelengths being different from each of the first and second useful signal wavelengths, the first and second useful infrared signal sensors (92, 100) are arranged such that their respective useful signal sensor sensing surfaces (92a, 100a) are symmetrically oriented with respect to a useful signal sensor symmetry plane (W) located between the useful signal sensor sensing surfaces (92a, 100a), and wherein the first and second infrared reference signal sensors (96, 104) are arranged such that their respective reference signal sensor sensing surfaces (96a, 104a) are symmetrically oriented with respect to a reference signal sensor symmetry plane (V) located between the reference signal sensor sensing surfaces (96a, 104a), wherein no useful signal sensor sensing surface (92a, 100a) is oriented orthogonally to the useful signal sensor symmetry plane (W) and wherein no reference signal sensor sensing surface (96a, 104a) is oriented orthogonally to the reference signal sensor symmetry plane (V),
**characterized in that,** the first infrared useful signal sensor (92) and the second infrared useful signal sensor (100) are arranged such that their respective sensor sensing surfaces (92a, 100a) are tilted towards each other about a virtual useful signal tilt axis (M), and the first infrared reference signal sensor (96) and the second infrared reference signal sensor (104) are arranged such that their respective reference sensor sensing surfaces (96a, 104a) are tilted towards each other about a virtual reference signal tilt axis (N) different from the useful signal tilt axis (M).

2. Multi-channel infrared gas sensor (54) according to Claim 1,
**characterized in that** the useful signal sensor symmetry plane (W) and the reference signal sensor symmetry plane (V) differ from one another.

3. A multi-channel infrared gas sensor (54) according to Claim 1 or 2,
**characterized in that** the beam splitter assembly (84) is a reflector beam splitter assembly and that all infrared useful signal sensors (92, 100) as well as all infrared reference signal sensors (96, 104) are located on an irradiation side of the beam splitter assembly (84) on which an infrared beam (64) entering the gas sensor (54) is incident on the beam splitter assembly (84).

4. A multi-channel infrared gas sensor (54) according to Claim 3,
**characterized in that** the infrared useful signal sensors (92, 100) and the infrared reference signal sensors (96, 104) are arranged alternately in the circumferential direction about a virtual arrangement axis (A).

5. Multi-channel infrared gas sensor (54) according to Claim 4,
**characterized in that** the first and the second infrared useful signal sensor (92, 100) are diametrically opposite to one another with respect to the virtual arrangement axis (A) and/or **in that** the first and the second infrared reference signal sensor (96, 104) are diametrically opposite to one another with respect to the virtual arrangement axis (A).

6. Multi-channel infrared gas sensor (54) according to Claim 4 or 5,
**characterized in that** the virtual arrangement axis (A) is the irradiation axis (E).

7. Multi-channel infrared gas sensor (54) according to one of Claims 4 to 6,
**characterized in that** the useful signal sensor symmetry plane (W) and/or the reference signal sensor symmetry plane (V) contains or contain the arrangement axis (A) or runs parallel to the arrangement axis (A).

8. A multi-channel infrared gas sensor (54) according to any one of the preceding claims,
**characterized in that** the sensor detecting surface (92a, 100a) of each useful infrared signal sensor (92, 100) and the sensor sensing surface (96a, 104a) of each reference infrared signal sensor (96, 104) is inclined towards the beam splitter arrangement (84).

9. A multi-channel infrared gas sensor (54) according to any one of the preceding claims,
**characterized in that** the first and/or the second useful signal wavelength is/are quantitatively located between the first and the second reference signal wavelength.

10. A multi-channel infrared gas sensor (54) according to any one of the preceding claims,
**characterized in that** the first and second useful signal wavelengths differ in amount by no more than one third of the smaller of the first and third bandwidths.

11. A multi-channel infrared gas sensor (54) according to any one of the preceding claims,
**characterized in that** the first and second useful signal wavelengths differ in amount by no more than one third of the smaller of the first and third bandwidths.

12. A multi-channel infrared gas sensor (54) according to one of the preceding claims,
**characterized in that** the gas sensor (54) comprises an evaluation device (14) which determines, from the signals of the first and the second infrared reference signal sensor (96, 104), reference information (136), which determines, from the signals of the first and the second infrared useful signal sensor (92, 100), useful information (132), and which outputs, from a comparison of reference information (136) and useful information (132), information about a proportion of a gas identified by the first and/or the second useful signal wavelength in a measurement gas irradiated with the incoming infrared beam (64).

13. A multi-channel infrared gas sensor (54) according to any one of the preceding claims,
**characterized in that** the gas sensor (54) comprises a sensor housing (66) having a first compartment (68) in which the beam splitter arrangement (84), the infrared useful signal sensors (92, 100) and the infrared reference signal sensors (96) are arranged, and with a second compartment (72) which is spatially remote from the first compartment (68) and in which an infrared radiation source (74) is arranged, wherein a receiving formation (79) for receiving a measuring cuvette (52) is arranged between the first and the second 15 compartment (68, 72).

14. A ventilator (10) for at least assisting artificial respiration of a living patient, comprising:
- a source of respiratory gas (12),
- a ventilation line arrangement (20) for conducting inspiratory ventilation gas from the ventilation gas source (12) to a patient-side proximal ventilation gas outlet opening (62) and for conducting expiratory ventilation gas away from a proximal ventilation gas inlet opening (62),
- a pressure changing device (13) for changing the pressure of the ventilation gas in the ventilation line arrangement (20),
- a control device (14) for operating the ventilation gas source (12) and/or the pressure changing device (13), and
- A multi-channel infrared gas sensor (54) according to one of the preceding claims for detecting at least one gas constituent in the inspiratory and/or expiratory ventilation gas.

15. A ventilation device (10) according to Claim 14,
**characterized in that** the multi-channel infrared gas sensor (54) is a gas sensor (54) according to Claim 11, wherein the evaluation device (14) is part of the control device (14) on the ventilation device (10).

## Revendications

1. Capteur de gaz infrarouge à canaux multiples (54), comprenant
- un dispositif diviseur de faisceau (84) qui est conçu pour diviser en une pluralité de faisceaux partiels infrarouges (85, 86, 87, 88) un faisceau infrarouge (64) incident sur le dispositif diviseur de faisceau (84) le long d'un axe d'incidence de faisceau (E) prédéterminé,
- un premier filtre passe-bande (90) disposé dans un premier trajet de faisceau d'un premier faisceau partiel infrarouge (85) avec une première largeur de bande prédéterminée et avec un maximum de transmission à une première longueur d'onde de signal utile prédéterminée,
- un premier capteur de signal utile infrarouge (92) disposé dans le premier trajet de faisceau du premier faisceau partiel infrarouge (85) derrière le premier filtre passe-bande (90),
- un deuxième filtre passe-bande (94), qui est disposé dans un deuxième trajet de faisceau d'un deuxième faisceau partiel infrarouge (86) différent du premier faisceau partiel infrarouge (58), dans lequel le deuxième filtre passe-bande (94) présente une deuxième largeur de bande prédéterminée et un maximum de transmission à une première longueur d'onde de signal de référence prédéterminée, dans lequel la première longueur d'onde de signal de référence est différente de la première longueur d'onde de signal utile,
- un premier capteur de signal de référence infrarouge (96) situé dans le deuxième trajet de faisceau du deuxième faisceau partiel infrarouge (86) derrière le deuxième filtre passe-bande (94),
dans lequel le dispositif diviseur de faisceau (84) est conçu pour diviser le faisceau infrarouge entrant (64) en au moins quatre faisceaux partiels infrarouges (85, 86, 87, 88), dans lequel le capteur de gaz infrarouge multicanal (54) comprend en outre :
- un troisième filtre passe-bande (98) disposé dans un troisième trajet de faisceau d'un troisième faisceau partiel infrarouge (87), dans lequel le troisième filtre passe-bande (98) présente une troisième largeur de bande prédéterminée et un maximum de transmission à une deuxième longueur d'onde de signal utile prédéterminée,
- un deuxième capteur de signal utile infrarouge (100) situé dans le troisième trajet de faisceau du troisième faisceau partiel infrarouge (87) derrière le troisième filtre passe-bande (98),
- un quatrième filtre passe-bande (102) disposé dans un quatrième trajet de faisceau d'un quatrième faisceau partiel infrarouge (88), dans lequel le quatrième filtre passe-bande (102) a une quatrième largeur de bande prédéterminée et un maximum de transmission à une deuxième longueur d'onde de signal de référence prédéterminée,
- un deuxième capteur de signal de référence infrarouge (104) situé dans le quatrième trajet de faisceau du quatrième faisceau partiel infrarouge (88) derrière le quatrième filtre passe-bande (102),
dans lequel les directions de trajet (85v, 86, 87v, 88v) des premier, deuxième, troisième et quatrième trajets de faisceau sont différentes les unes des autres par paires, chaque longueur d'onde desdites première et deuxième longueurs d'onde de signal de référence étant différente de chaque longueur d'onde desdites première et deuxième longueurs d'onde de signal utile, dans lequel lesdits premier et deuxième capteurs de signal utile infrarouge (92, 100) sont agencés de telle sorte que leurs surfaces de détection de capteur de signal utile respectives (92a, 100a) sont orientées symétriquement par rapport à un plan de symétrie de capteur de signal utile (W) situé entre les surfaces de détection de capteur de signal utile (96a, 104a), et dans lequel les premier et deuxième capteurs de signal de référence infrarouge (96, 104) sont disposés de telle sorte que leurs surfaces de détection de capteur de signal de référence respectives (96a, 104a) sont orientées symétriquement par rapport à un plan de symétrie de capteur de signal de référence (V) situé entre les surfaces de détection de capteur de signal de référence (96a, 104a), dans lequel aucune surface de détection de capteur de signal utile (92a, 100a) n'est orientée orthogonalement au plan de symétrie de capteur de signal utile (W) et dans lequel aucune surface de détection de capteur de signal de référence (96a, 1004a) n'est orientée orthogonalement au plan de symétrie de capteur de signal de référence (V),
**caractérisé en ce que** le premier capteur de signal utile infrarouge (92) et le deuxième capteur de signal utile infrarouge (100) sont disposés de telle sorte que leurs surfaces de détection de capteur de signal utile respectives (92a, 100a) sont inclinées l'une vers l'autre autour d'un axe d'inclinaison de signal utile virtuel (M), et **en ce que** le premier capteur de signal de référence infrarouge (96) et le deuxième capteur de signal de référence infrarouge (104) sont disposés de telle sorte que leurs surfaces de détection de capteur de signal de référence respectives (96a, 104a) sont inclinées l'une vers l'autre autour d'un axe d'inclinaison de signal de référence virtuel (N) différent de l'axe d'inclinaison de signal utile (M).

2. Capteur de gaz infrarouge multicanal (54) selon la revendication 1,
**caractérisé en ce que** le plan de symétrie de capteur de signal utile (W) et le
plan de symétrie du capteur de signal de référence (V) sont différents l'un de l'autre.

3. Capteur de gaz infrarouge multicanal (54) selon la revendication 1 ou 2,
**caractérisé en ce que** le dispositif diviseur de faisceau (84) est un dispositif diviseur de faisceau à réflecteur et **en ce que** tous les capteurs de signal utile infrarouge (92, 100) ainsi que tous les capteurs de signal de référence infrarouge (96, 104) se trouvent sur un côté de rayonnement du dispositif diviseur de faisceau (84), sur lequel un faisceau infrarouge (64) entrant dans le capteur de gaz (54) rayonne sur le dispositif diviseur de faisceau (84).

4. Capteur de gaz infrarouge multicanal (54) selon la revendication 3,
**caractérisé en ce que** les capteurs de signal utile infrarouge (92, 100) et les capteurs de signal infrarouge de référence (96, 104) sont disposés en alternance dans la direction circonférentielle autour d'un axe d'agencement virtuel (A).

5. Capteur de gaz infrarouge multicanal (54) selon la revendication 4,
**caractérisé en ce que** le premier et le deuxième capteur de signal utile infrarouge (92, 100) sont diamétralement opposés l'un à l'autre par rapport à l'axe d'agencement virtuel (A) ou/et **en ce que** le premier et le deuxième capteur de signal infrarouge de référence (96, 104) sont diamétralement opposés l'un à l'autre par rapport à l'axe d'agencement virtuel (A).

6. Capteur de gaz infrarouge multicanal (54) selon la revendication 4 ou 5,
**caractérisé en ce que** l'axe d'agencement virtuel (A) est l'axe d'incidence de faisceau (E).

7. Capteur de gaz infrarouge multicanal (54) selon l'une des revendications 4 à 6, **caractérisé en ce que** le plan de symétrie de capteur de signal utile (W) ou/et le plan de symétrie du capteur de signal de référence (V) contient/contiennent l'axe d'agencement (A) ou est/sont parallèles à l'axe d'agencement (A).

8. Capteur de gaz infrarouge multicanal (54) selon l'une des revendications précédentes,
**caractérisé en ce que** la surface de détection de capteur (92a, 100a) de chaque capteur de signal utile infrarouge (92, 100) et la surface de détection de capteur (96a, 104a) de chaque capteur de signal de référence infrarouge (96, 104) sont inclinées vers le dispositif diviseur de faisceau (84).

9. Capteur de gaz infrarouge multicanal (54) selon l'une des revendications précédentes,
**caractérisé en ce que** la première ou/et la deuxième longueur d'onde du signal utile est/sont situées en valeur absolue entre la première et la deuxième longueur d'onde du signal de référence.

10. Capteur de gaz infrarouge multicanal (54) selon l'une des revendications précédentes,
**caractérisé en ce que** la première et la deuxième longueur d'onde du signal utile ne diffèrent pas en valeur absolue de plus d'un tiers de la largeur de bande la plus petite en valeur absolue entre la première et la troisième largeur de bande.

11. Capteur de gaz infrarouge multicanal (54) selon l'une des revendications précédentes,
**caractérisé en ce que** chaque largeur de bande des deuxième et quatrième largeurs de bande est inférieure en valeur absolue à chaque largeur de bande des première et troisième largeurs de bande.

12. Capteur de gaz infrarouge multicanal (54) selon l'une des revendications précédentes,
**caractérisé en ce que** le capteur de gaz (54) comprend un dispositif d'évaluation (14) qui détermine une information de référence (136) à partir des signaux du premier et du deuxième capteur de signal infrarouge de référence (96, 104), qui détermine à partir des signaux du premier et du deuxième capteur de signal utile infrarouge (92, 100) une information utile (132) et qui, à partir d'une comparaison entre l'information de référence (136) et l'information utile (132), délivre une information sur une proportion d'un gaz identifié par la première ou/et la deuxième longueur d'onde du signal utile dans un gaz de mesure irradié par le faisceau infrarouge (64) entrant.

13. Capteur de gaz infrarouge multicanal (54) selon l'une des revendications précédentes,
**caractérisé en ce que** le capteur de gaz (54) présente un boîtier de capteur (66) avec un premier compartiment (68), dans lequel sont disposés le dispositif diviseur de faisceau (84), les capteurs de signaux utiles infrarouges (92, 100) et les capteurs de signaux infrarouges de référence (96), et avec un deuxième compartiment (72) éloigné dans l'espace du premier compartiment (68), dans lequel est disposée une source de rayonnement infrarouge (74), dans lequel une formation de réception (79) est disposée entre le premier et le deuxième compartiment (68, 72) pour recevoir une cuvette de mesure (52) entre le premier et le deuxième compartiment (68, 72).

14. Dispositif de ventilation (10) pour la ventilation artificielle au moins assistée d'un patient vivant, comprenant :
- une source de gaz respiratoire (12),
- un agencement de conduit de ventilation (20) pour conduire le gaz de ventilation inspiratoire de la source de gaz de ventilation (12) à un orifice de sortie de gaz de ventilation proximal (62) du côté du patient et pour conduire le gaz de ventilation expiratoire à l'écart d'un orifice d'entrée de gaz de ventilation proximal (62),
- un dispositif de modification de la pression (13) pour modifier la pression du gaz respiratoire dans l'ensemble de conduits respiratoires (20),
- un dispositif de commande (14) pour le fonctionnement de la source de gaz respiratoire (12) ou/et du dispositif de modification de la pression (13) ainsi que
- un capteur de gaz infrarouge multicanal (54) selon l'une des revendications précédentes pour détecter au moins un composant gazeux dans le gaz respiratoire inspiratoire ou/et expiratoire.

15. Dispositif de ventilation (10) selon la revendication 14,
**caractérisé en ce que** le capteur de gaz infrarouge multicanal (54) est un capteur de gaz (54) selon la revendication 11, le dispositif d'évaluation (14) faisant partie du dispositif de commande (14) du dispositif de ventilation (10).
